# EUROPEAN PATENT APPLICATION

(11) **EP 4 703 363 A1**
(43) Date of publication of application: **04.03.2026**
(21) Application number: 24796201.2
(22) Date of filing: 26.04.2024
(51) Int. Cl.: C07D 495/04, A61K 31/519, A61P 15/08, A61P 15/00

(54) **SALT FORM AND CRYSTAL FORM OF THIENOPYRIMIDINE COMPOUND, AND PREPARATION METHOD THEREFOR AND USE THEREOF**

(30) Priority: 27.04.2023 CN 202310498157
(71) Applicant: Shandong Luye Pharmaceutical Co., Ltd., Yantai, Shandong 264670 (CN)
(72) Inventor: WU, Yanhua, Yantai, Shandong 264670 (CN); ZHAO, Jiye, Yantai, Shandong 264670 (CN); WANG, Guanghui, Yantai, Shandong 264670 (CN)
(74) Representative: karo IP
(86) International application number: PCT/CN2024/089932
(87) International publication number: WO 2024/222827

(57) **Abstract**

The present disclosure relates to a salt form and crystal form of a thienopyrimidine compound, and a preparation method therefor and the use thereof. Specifically, provided are a salt form and crystal form of the compound of formula I, a preparation method therefor, and the use thereof in the preparation of a drug as a GnRH receptor antagonist.

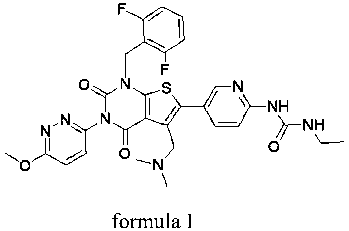

## Description

### TECHNICAL FIELD

The present disclosure pertains to the field of pharmaceutical chemistry, and particularly relates to salt forms and crystal forms of a thienopyrimidine compound, a preparation method therefor, and use thereof.

### BACKGROUND

The secretion of anterior pituitary hormones is feedback-controlled by peripheral hormones secreted from the target organs of the corresponding hormones and by secretion-regulation hormones from the hypothalamus, which is the upper central organ of the anterior pituitary (these hormones are collectively referred to as "hypothalamic hormones"). Currently, nine hormones, including thyrotropin-releasing hormone (TRH) and gonadotropin-releasing hormone (GnRH), have been shown to be hypothalamic hormones. These hypothalamic hormones are thought to exhibit their effects via receptors that are thought to be present in the anterior pituitary. Efforts have been made to find receptor-gene expression specific to these hormones. Thus, antagonists or agonists that act specifically and selectively on these receptors should control the action of hypothalamic hormones and the secretion of anterior pituitary hormones. Therefore, such antagonists or agonists are expected to be useful for preventing or treating anterior pituitary hormone-dependent diseases.

As agents for inhibiting GnRH receptors and the function of GnRH receptors, superagonists have been used as agents for treating sex hormone-dependent diseases such as prostate cancer, breast cancer, and endometriosis. GnRH receptor superagonists bind to the GnRH receptor and exert an initial transient gonadotropin secretion-stimulating effect, a so-called "flare-up" phenomenon, and then inhibit the function by inducing a depletion of the gonadotropin and inhibiting the down-regulation of the GnRH receptor. Therefore, since GnRH receptor superagonists initially promote the secretion of gonadotropins, there is a defect that the disease is transiently worsened. In contrast, the inhibitory mechanism of GnRH receptor antagonists (hereinafter referred to as "GnRH antagonists") is the inhibition of binding to the GnRH receptor; therefore, they are expected to exert an inhibitory effect rapidly without gonadotropin secretion.

There are many problems to be solved with peptide compounds, and they are associated with oral absorbability, dosage form, dose volume, drug stability, sustained action, metabolic stability, etc. In light of the limitations of peptidic GnRH receptor antagonists, some non-peptidic GnRH receptor antagonists have been proposed and are in the development, clinical trial, or marketing stage. For example, Elagolix (also known as NBI-56418 or ABT-620), a small-molecule GnRH receptor antagonist cooperatively developed by Abbott and Neurocrine Biosciences Inc., is currently in the clinical Phase III stage and is mainly used in the treatment of endometriosis (Phase III) and hysteromyoma (Phase II).

Relugolix, also known as TAK-385, is an oral small-molecule antagonist of the GnRH receptor developed by Takada Pharmaceutical in Japan, and is used for treating endometriosis, hysteromyoma, and prostate cancer.

PCT/CN2022/128588 provides a GnRH receptor antagonist having a structural formula as shown below. Test results show that the compound has significant inhibitory and binding effects on the GnRH receptor and has good pharmacokinetic absorption and high bioavailability. The application is incorporated herein in its entirety.

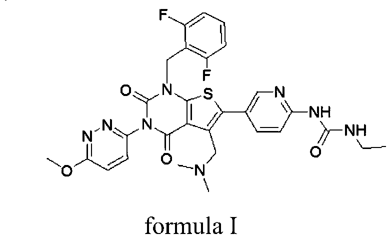

The pharmaceutical form (e.g., a crystal form or salt) of a compound often affects the chemical stability of the drug. Different crystallization conditions and storage conditions may lead to changes in the crystal form structure of the compound, sometimes accompanied by the foumation of other crystal forms. Generally, amorphous drug products have no regular crystal form structure and often have defects such as poor product stability, fine crystals, difficult filtration, proneness to agglomeration, and poor flowability. In light of the importance of solid drug salt forms and crystal forms and their stability in clinical treatment, the in-depth research on salt forms and crystal forms of the compound of formula I is of great significance for developing drugs that are suitable for industrial production and have good biological activity.

### SUMMARY

In one aspect, the present disclosure provides a crystal form A of the compound of formula I, having, in an X-ray powder diffraction pattern using Cu-Kα radiation, characteristic diffraction peaks at the following 2θ angles: 7.2390 ± 0.2°, 8.7232 ± 0.2°, 10.5072 ± 0.2°, 12.4670 ± 0.2°, 18.6162 ± 0.2°, 19.6161 ± 0.2°, and 22.1387 ± 0.2°.

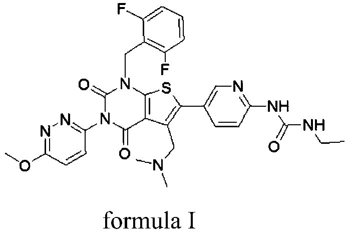

In some embodiments, the crystal form A of the compound of formula I has, in an X-ray powder diffraction pattern using Cu-Kα radiation, characteristic diffraction peaks at the following 2θ angles: 7.2390 ± 0.2°, 8.7232 ± 0.2°, 10.5072 ± 0.2°, 10.9392 ± 0.2°, 12.4670 ± 0.2°, 17.6527 ± 0.2°, 18.6162 ± 0.2°, 19.6161 ± 0.2°, 22.1387 ± 0.2°, 23.4047 ± 0.2°, and 27.3319 ± 0.2°.

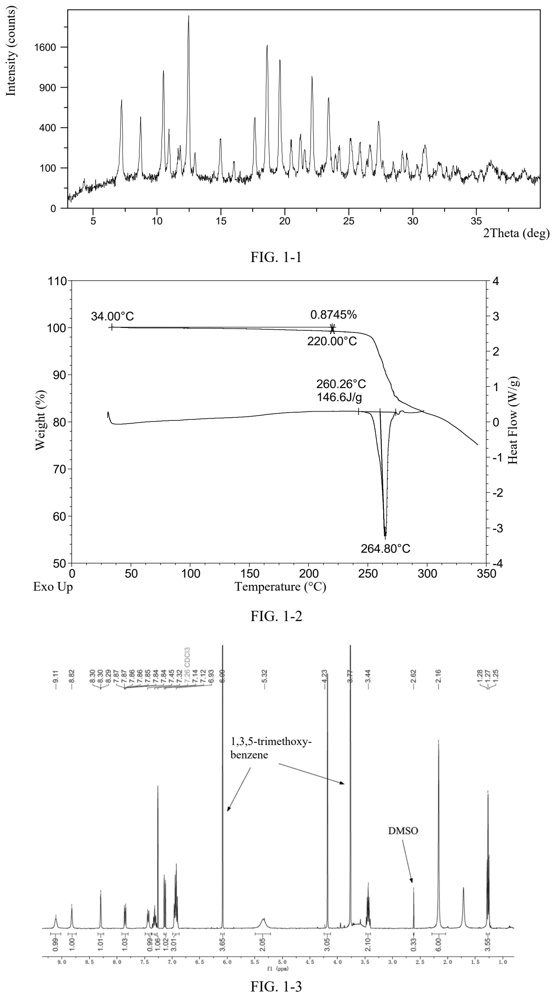

In some embodiments, the crystal form A of the compound of formula I has an X-ray powder diffraction pattern using Cu-Kα radiation as shown in FIG. 1-1.

In one aspect, the present disclosure provides a pharmaceutically acceptable salt of the compound of formula I, wherein the pharmaceutically acceptable salt is selected from the group consisting of a hydrochloride, sulfate, phosphate, maleate, tartrate, fumarate, citrate, malate, hippurate, lactate, succinate, acetate, p-toluenesulfonate, methanesulfonate, benzenesulfonate, oxalate, malonate, gentisate, or benzoate.

In some embodiments, the pharmaceutically acceptable salt is a hydrochloride, fumarate, L-malate, methanesulfonate, benzenesulfonate, or gentisate.

In some embodiments, in the pharmaceutically acceptable salt, the molar ratio of the compound of formula I to an acid molecule is about 1:2 to 2:1, preferably about 1:2, 1:1, or 2:1, and more preferably about 1:1.

The present disclosure further provides a preparation method for the pharmaceutically acceptable salt of the compound of formula I, comprising a step of mixing the compound of formula I with an acid and a solvent. The solvent is selected from the group consisting of one or more of methanol, dichloromethane, ethyl acetate, acetone, tetrahydrofuran, and acetonitrile.

In some embodiments, the molar ratio of the feeding amount of the compound of formula I to the feeding amount of the acid is selected from the group consisting of 5:1 to 1:5, preferably 2:1 to 1:2, and more preferably 2:1 or 1:1.

In one aspect, the present disclosure provides a crystal form A of a fumarate of the compound of formula I, having, in an X-ray powder diffraction pattern using Cu-Kα radiation, characteristic diffraction peaks at the following 2θ angles: 6.9355 ± 0.2°, 9.6549 ± 0.2°, 14.4518 ± 0.2°, 18.8612 ± 0.2°, 20.2529 ± 0.2°, and 22.9352 ± 0.2°.

In some embodiments, the crystal form A of the fumarate of the compound of formula I has, in an X-ray powder diffraction pattern using Cu-Kα radiation, characteristic diffraction peaks at the following 20 angles: 6.9355 ± 0.2°, 9.3706 ± 0.2°, 9.6549 ± 0.2°, 9.8505 ± 0.2°, 12.4723 ± 0.2°, 14.4518 ± 0.2°, 17.2206 ± 0.2°, 18.8612 ± 0.2°, 20.2529 ± 0.2°, 20.5609 ± 0.2°, 21.1589 ± 0.2°, 22.2396 ± 0.2°, 22.5989 ± 0.2°, 22.9352 ± 0.2°, and 26.2998 ± 0.2°.

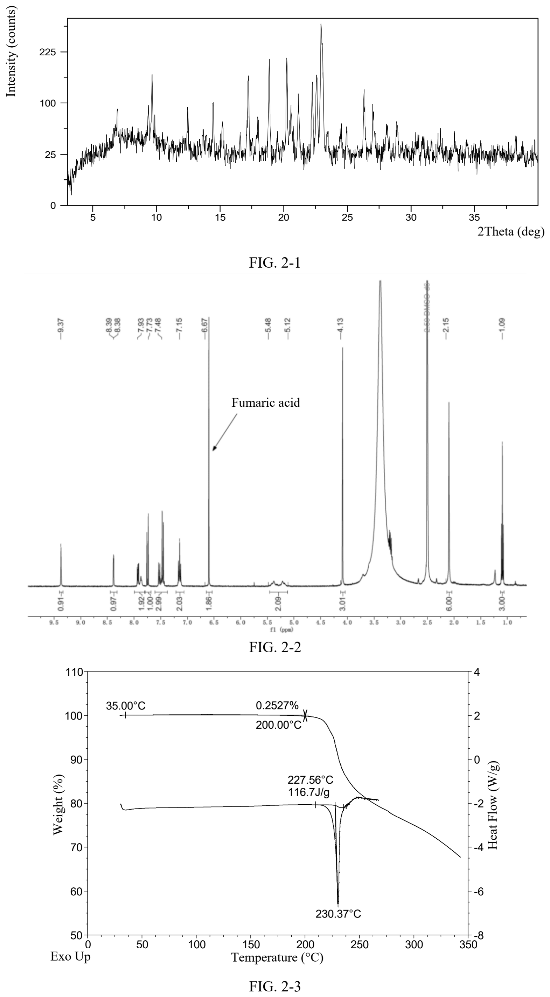

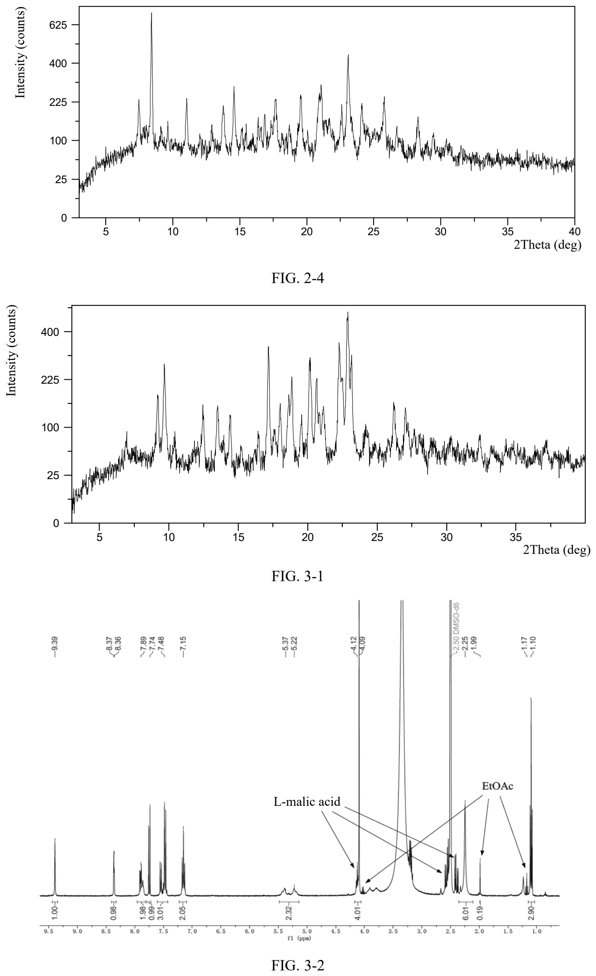

In some embodiments, the crystal form A of the fumarate of the compound of formula I has an X-ray powder diffraction pattern using Cu-Kα radiation as shown in FIG. 2-1.

The present disclosure provides a preparation method for the crystal form A of the fumarate of the compound of formula I, comprising steps of mixing the compound of formula I, fumaric acid, and a solvent, and crystallization. In an optional embodiment, the solvent is methanol-dichloromethane.

In one aspect, the present disclosure provides a crystal form B of a fumarate of the compound of formula I, having, in an X-ray powder diffraction pattern using Cu-Kα radiation, characteristic diffraction peaks at the following 2θ angles: 7.4623 ± 0.2°, 8.4044 ± 0.2°, 11.0463 ± 0.2°, 13.7771 ± 0.2°, 14.5639 ± 0.2°, and 23.0754 ± 0.2°.

In some embodiments, the crystal form B of the fumarate of the compound of formula I has, in an X-ray powder diffraction pattern using Cu-Kα radiation, characteristic diffraction peaks at the following 20 angles: 7.4623 ± 0.2°, 8.4044 ± 0.2°, 11.0463 ± 0.2°, 13.7771 ± 0.2°, 14.5639 ± 0.2°, 17.6799 ± 0.2°, 19.5247 ± 0.2°, 20.9439 ± 0.2°, 21.1012 ± 0.2°, 23.0754 ± 0.2°, 24.1136 ± 0.2°, and 25.8680 ± 0.2°.

In some embodiments, the crystal form B of the fumarate of the compound of formula I has an X-ray powder diffraction pattern using Cu-Kα radiation as shown in FIG. 2-4.

In one aspect, the present disclosure provides a crystal form A of an L-malate of the compound of formula I, having, in an X-ray powder diffraction pattern using Cu-Kα radiation, characteristic diffraction peaks at the following 2θ angles: 9.2046 ± 0.2°, 9.6751 ± 0.2°, 12.4714 ± 0.2°, 13.5073 ± 0.2°, 17.1769 ± 0.2°, 20.1444 ± 0.2°, and 22.8785 ± 0.2°.

In some embodiments, the crystal form A of the L-malate of the compound of formula I has, in an X-ray powder diffraction pattern using Cu-Kα radiation, characteristic diffraction peaks at the following 2θ angles: 9.2046 ± 0.2°, 9.6751 ± 0.2°, 12.4714 ± 0.2°, 13.5073 ± 0.2°, 14.4312 ± 0.2°, 17.1769 ± 0.2°, 18.0032 ± 0.2°, 18.6031 ± 0.2°, 18.8495 ± 0.2°, 20.1444 ± 0.2°, 20.6407 ± 0.2°, 22.2793 ± 0.2°, 22.8785 ± 0.2°, 23.1632 ± 0.2°, 26.2217 ± 0.2°, and 27.0313 ± 0.2°.

In some embodiments, the crystal form A of the L-malate of the compound of formula I has an X-ray powder diffraction pattern using Cu-Kα radiation as shown in FIG. 3-1.

The present disclosure provides a preparation method for the crystal form A of the L-malate of the compound of formula I, comprising steps of mixing the compound of formula I, L-malic acid, and a solvent, and crystallization. In an optional embodiment, the solvent is ethyl acetate.

In one aspect, the present disclosure provides a crystal form A of a hydrochloride of the compound of formula I, having, in an X-ray powder diffraction pattern using Cu-Kα radiation, characteristic diffraction peaks at the following 2θ angles: 9.1252 ± 0.2°, 10.4351 ± 0.2°, 11.2797 ± 0.2°, and 12.9108 ± 0.2°.

In some embodiments, the crystal form A of the hydrochloride of the compound of formula I has, in an X-ray powder diffraction pattern using Cu-Kα radiation, characteristic diffraction peaks at the following 2θ angles: 9.1252 ± 0.2°, 9.6045 ± 0.2°, 10.4351 ± 0.2°, 11.2797 ± 0.2°, 12.9108 ± 0.2°, 17.0513 ± 0.2°, 17.5138 ± 0.2°, 20.9172 ± 0.2°, 21.6060 ± 0.2°, and 26.0138 ± 0.2°.

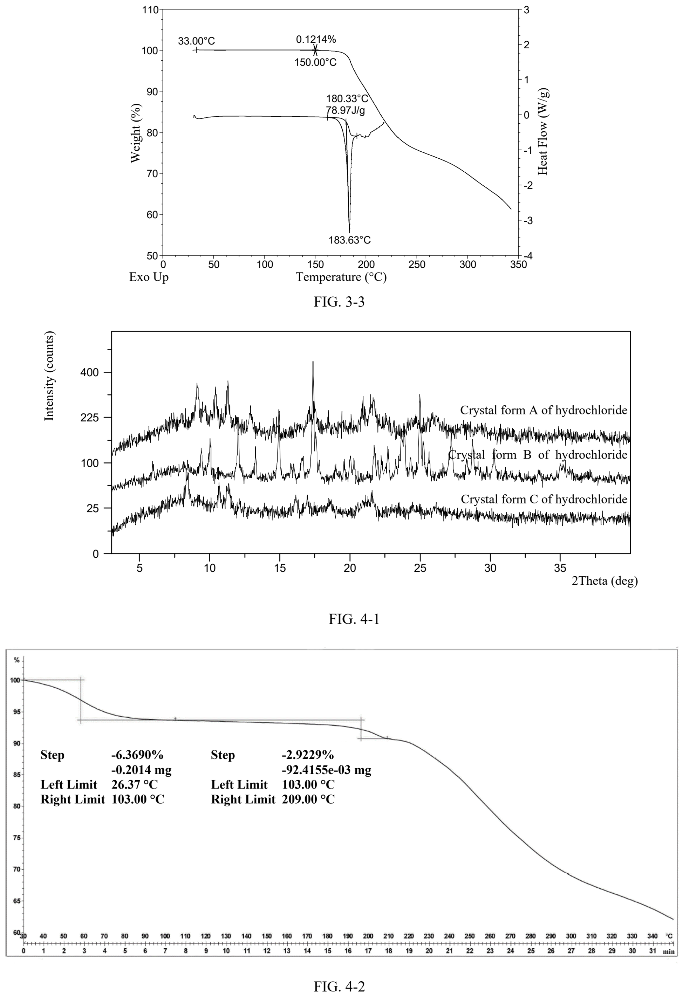

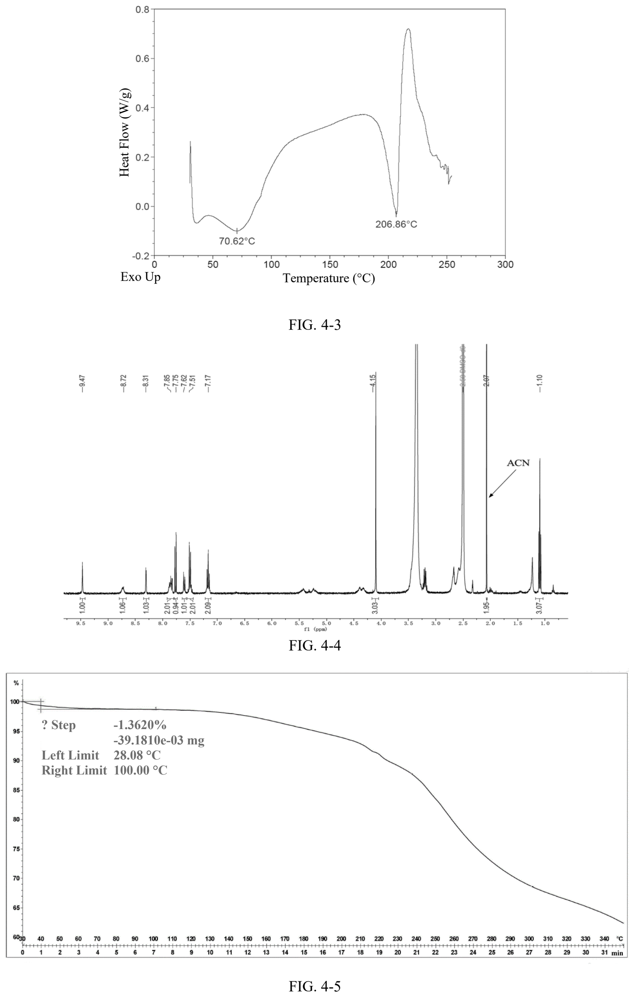

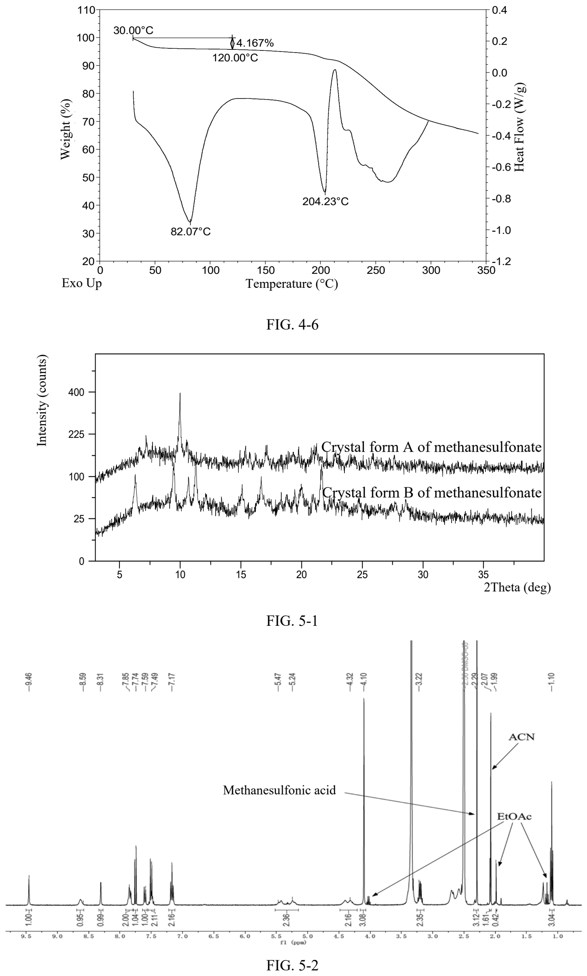

In some embodiments, the crystal form A of the hydrochloride of the compound of formula I has an X-ray powder diffraction pattern using Cu-Kα radiation as shown in FIG. 4-1.

The present disclosure provides a preparation method for the crystal form A of the hydrochloride of the compound of formula I, comprising steps of mixing the compound of formula I, hydrochloric acid, and a solvent, and crystallization. In an optional embodiment, the solvent is acetone, ethyl acetate, or tetrahydrofuran-water.

In one aspect, the present disclosure provides a crystal form B of a hydrochloride of the compound of formula I, having, in an X-ray powder diffraction pattern using Cu-Kα radiation, characteristic diffraction peaks at the following 2θ angles: 9.4060 ± 0.2°, 10.0584 ± 0.2°, 12.0366 ± 0.2°, 14.9457 ± 0.2°, 17.3666 ± 0.2°, and 24.9835 ± 0.2°.

In some embodiments, the crystal form B of the hydrochloride of the compound of formula I has, in an X-ray powder diffraction pattern using Cu-Kα radiation, characteristic diffraction peaks at the following 2θ angles: 9.4060 ± 0.2°, 10.0584 ± 0.2°, 12.0366 ± 0.2°, 13.2712 ± 0.2°, 14.9457 ± 0.2°, 17.3666 ± 0.2°, 17.5949 ± 0.2°, 23.6400 ± 0.2°, 23.8466 ± 0.2°, 24.9835 ± 0.2°, and 27.2387 ± 0.2°.

In some embodiments, the crystal form B of the hydrochloride of the compound of formula I has an X-ray powder diffraction pattern using Cu-Kα radiation as shown in FIG. 4-1.

The present disclosure provides a preparation method for the crystal form B of the hydrochloride of the compound of formula I, comprising steps of mixing the compound of formula I, hydrochloric acid, and a solvent, and crystallization. In an optional embodiment, the solvent is acetonitrile.

In one aspect, the present disclosure provides a crystal form C of a hydrochloride of the compound of formula I, having, in an X-ray powder diffraction pattern using Cu-Kα radiation, characteristic diffraction peaks at the following 2θ angles: 8.3817 ± 0.2°, 10.6700 ± 0.2°, 11.3268 ± 0.2°, and 16.1577 ± 0.2°.

In some embodiments, the crystal form C of the hydrochloride of the compound of formula I has, in an X-ray powder diffraction pattern using Cu-Kα radiation, characteristic diffraction peaks at the following 2θ angles: 8.3817 ± 0.2°, 10.6700 ± 0.2°, 11.3268 ± 0.2°, 16.1577 ± 0.2°, 16.9678 ± 0.2°, 18.5478 ± 0.2°, and 21.5630 ± 0.2°.

In some embodiments, the crystal form C of the hydrochloride of the compound of formula I has an X-ray powder diffraction pattern using Cu-Kα radiation as shown in FIG. 4-1.

The present disclosure provides a preparation method for the crystal form C of the hydrochloride of the compound of formula I, comprising steps of mixing the compound of formula I, hydrochloric acid, and a solvent, and crystallization. In an optional embodiment, the solvent is methanol-dichloromethane. In one aspect, the present disclosure provides a crystal form A of a methanesulfonate of the compound of formula I, having, in an X-ray powder diffraction pattern using Cu-Kα radiation, characteristic diffraction peaks at the following 2θ angles: 6.6779 ± 0.2°, 7.2085 ± 0.2°, 9.9783 ± 0.2°, and 10.5584 ± 0.2°.

In some embodiments, the crystal form A of the methanesulfonate of the compound of formula I has, in an X-ray powder diffraction pattern using Cu-Kα radiation, characteristic diffraction peaks at the following 2θ angles: 6.6779 ± 0.2°, 7.2085 ± 0.2°, 9.9783 ± 0.2°, 10.5584 ± 0.2°, 15.3689 ± 0.2°, 17.0799 ± 0.2°, 19.7556 ± 0.2°, and 21.0763 ± 0.2°.

In some embodiments, the crystal form A of the methanesulfonate of the compound of formula I has an X-ray powder diffraction pattern using Cu-Kα radiation as shown in FIG. 5-1.

The present disclosure provides a preparation method for the crystal form A of the methanesulfonate of the compound of formula I, comprising steps of mixing the compound of formula I, methanesulfonic acid, and a solvent, and crystallization. In an optional embodiment, the solvent is acetonitrile.

In one aspect, the present disclosure provides a crystal form B of a methanesulfonate of the compound of formula I, having, in an X-ray powder diffraction pattern using Cu-Kα radiation, characteristic diffraction peaks at the following 2θ angles: 6.2955 ± 0.2°, 9.4469 ± 0.2°, 10.6946 ± 0.2°, 11.2857 ± 0.2°, 16.6835 ± 0.2°, and 21.6727 ± 0.2°.

In some embodiments, the crystal form B of the methanesulfonate of the compound of formula I has, in an X-ray powder diffraction pattern using Cu-Kα radiation, characteristic diffraction peaks at the following 2θ angles: 6.2955 ± 0.2°, 9.4469 ± 0.2°, 10.6946 ± 0.2°, 11.2857 ± 0.2°, 12.1034 ± 0.2°, 15.0752 ± 0.2°, 16.6835 ± 0.2°, 18.7649 ± 0.2°, 19.4556 ± 0.2°, 19.9441 ± 0.2°, and 21.6727 ± 0.2°.

In some embodiments, the crystal form B of the methanesulfonate of the compound of formula I has an X-ray powder diffraction pattern using Cu-Kα radiation as shown in FIG. 5-1.

The present disclosure provides a preparation method for the crystal form B of the methanesulfonate of the compound of formula I, comprising steps of mixing the compound of formula I, methanesulfonic acid, and a solvent, and crystallization. In an optional embodiment, the solvent is acetone.

In one aspect, the present disclosure provides a crystal form A of a benzenesulfonate of the compound of formula I, having, in an X-ray powder diffraction pattern using Cu-Kα radiation, characteristic diffraction peaks at the following 2θ angles: 8.4849 ± 0.2°, 8.8939 ± 0.2°, 9.1410 ± 0.2°, 11.2190 ± 0.2°, 14.2050 ± 0.2°, 15.6291 ± 0.2°, 20.8604 ± 0.2°, and 23.7113 ± 0.2°.

In some embodiments, the crystal form A of the benzenesulfonate of the compound of formula I has, in an X-ray powder diffraction pattern using Cu-Kα radiation, characteristic diffraction peaks at the following 2θ angles: 8.4849 ± 0.2°, 8.8939 ± 0.2°, 9.1410 ± 0.2°, 11.2190 ± 0.2°, 14.2050 ± 0.2°, 15.6291 ± 0.2°, 19.2128 ± 0.2°, 19.9372 ± 0.2°, 20.8604 ± 0.2°, 21.5615 ± 0.2°, 23.7113 ± 0.2°, 24.0194 ± 0.2°, and 24.7649 ± 0.2°.

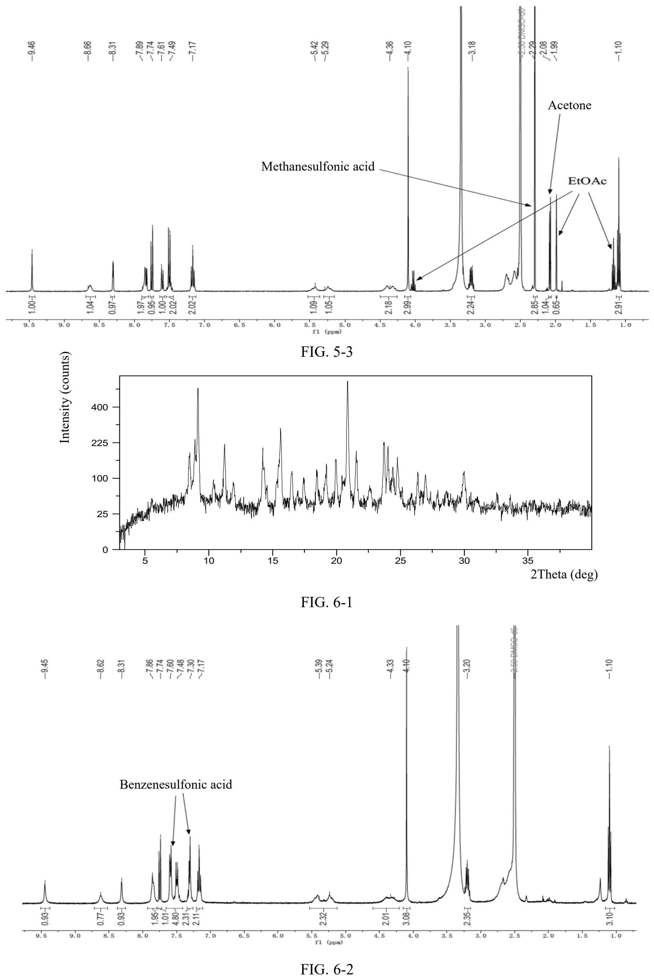

In some embodiments, the crystal form A of the benzenesulfonate of the compound of formula I has an X-ray powder diffraction pattern using Cu-Kα radiation as shown in FIG. 6-1.

The present disclosure provides a preparation method for the crystal form A of the benzenesulfonate of the compound of formula I, comprising steps of mixing the compound of formula I, benzenesulfonic acid, and a solvent, and crystallization. In an optional embodiment, the solvent is acetonitrile or ethyl acetate.

In one aspect, the present disclosure provides a crystal form A of a gentisate of the compound of formula I, having, in an X-ray powder diffraction pattern using Cu-Kα radiation, characteristic diffraction peaks at the following 2θ angles: 6.5498 ± 0.2°, 7.8186 ± 0.2°, 10.1923 ± 0.2°, 12.0696 ± 0.2°, 18.6218 ± 0.2°, and 25.8173 ± 0.2°.

In some embodiments, the present disclosure provides a crystal form A of a gentisate of the compound of formula I, having, in an X-ray powder diffraction pattern using Cu-Kα radiation, characteristic diffraction peaks at the following 2θ angles: 6.5498 ± 0.2°, 7.8186 ± 0.2°, 10.1923 ± 0.2°, 12.0696 ± 0.2°, 13.1877 ± 0.2°, 15.1362 ± 0.2°, 17.3902 ± 0.2°, 18.6218 ± 0.2°, 20.9685 ± 0.2°, 21.2453 ± 0.2°, 24.3675 ± 0.2°, 25.8173 ± 0.2°, 26.6389 ± 0.2°, and 27.7044 ± 0.2°.

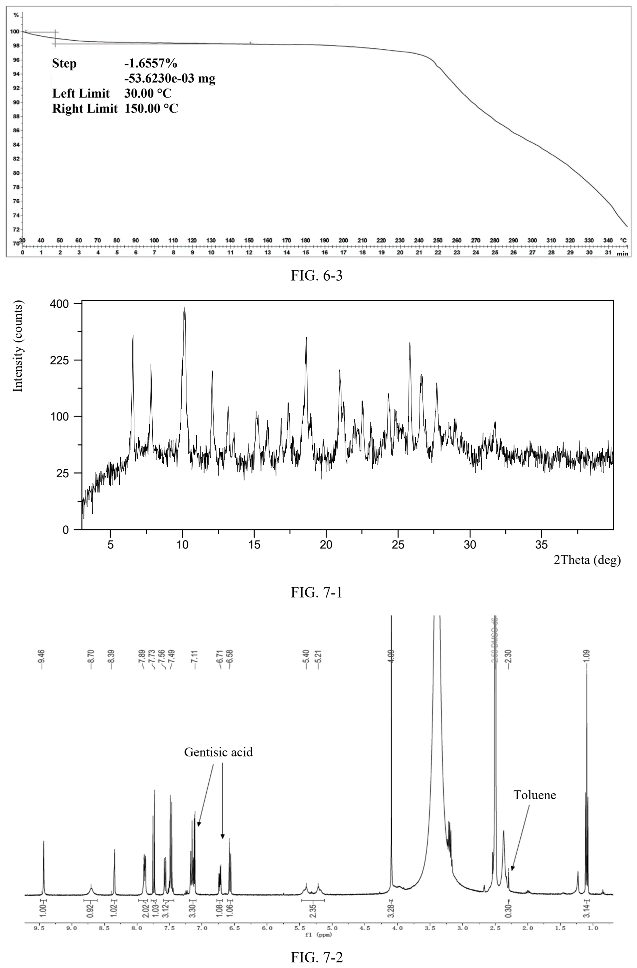

In some embodiments, the crystal form A of the gentisate of the compound of formula I has an X-ray powder diffraction pattern using Cu-Kα radiation as shown in FIG. 7-1.

The present disclosure provides a preparation method for the crystal form A of the gentisate of the compound of formula I, comprising steps of mixing the compound of formula I, gentisic acid, and a solvent, and crystallization. In an optional embodiment, the solvent is methanol-dichloromethane.

In an optional embodiment, in the preparation method for any one of the crystal forms described above, the crystallization method is selected from the group consisting of one or more of room temperature crystallization, cooling crystallization, solvent volatilization crystallization, anti-solvent addition crystallization, or seed crystal addition-induced crystallization.

The present disclosure further provides a pharmaceutical composition comprising the crystal form A of the compound of formula I, any one of the aforementioned pharmaceutically acceptable salts or crystal forms, and a pharmaceutically acceptable carrier. The pharmaceutical composition can be prepared into various pharmaceutically acceptable dosage forms, such as tablets, capsules, oral liquid, granules, injections, or various sustained and controlled release preparations. The pharmaceutical composition can be administered orally or parenterally (e.g., intravenously, subcutaneously, or topically). The administration dose may be suitably adjusted depending on the age, gender, and type of disease of a patient, and is generally about 1-200 mg per day.

In one aspect, the present disclosure further provides use of the crystal form A of the compound of formula I, any one of the aforementioned pharmaceutically acceptable salts or crystal forms, or the pharmaceutical composition in the preparation of a medicament of a GnRH receptor antagonist.

In one aspect, the present disclosure further provides use of the crystal form A of the compound of formula I, any one of the aforementioned pharmaceutically acceptable salts or crystal forms, or the pharmaceutical composition in the preparation of a medicament for preventing and/or treating a sex hormone-dependent disease. The sex hormone-dependent disease includes sex hormone-dependent cancer, bone metastasis of sex hormone-dependent cancer, prostatic hypertrophy, prostate cancer, hysteromyoma, endometriosis, metrofibroma, precocious puberty, amenorrhea, premenstrual syndrome, dysmenorrhoea, multilocular ovarian syndrome, polycystic ovary syndrome, acne, alopecia, Alzheimer's disease, infertility, irritable bowel syndrome, benign or malignant tumors that are hormone-independent and sensitive to LH-RH (luteinizing hormone-releasing hormone), or flushing. The sex hormone-dependent cancer is selected from the group consisting of prostate cancer, uterine cancer, breast cancer, or pituitary cancer.

In some embodiments of the present disclosure, the use is use of the crystal form A of the compound of formula I, any one of the aforementioned pharmaceutically acceptable salts or crystal forms, or the pharmaceutical composition as a reproduction modulator, a contraceptive drug, an ovulation inducer, or a medicament for preventing early ovulation, or as a medicament for preventing a postoperative recurrence of sex hormone-dependent cancer.

### Definitions and Description

Unless otherwise specified, the following terms and phrases used herein are intended to have the following meanings. A particular term or phrase, when not specifically defined, should not be considered indefinite or unclear, but should be construed according to its common meaning. When referring to a trade name, it is intended to refer to its corresponding commercial product or its active ingredient.

The term "pharmaceutically acceptable" is described in the present disclosure for the compounds, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The compounds of the present disclosure may have asymmetric carbon atoms (optical centers) or double bonds. Racemates, diastereoisomers, geometric isomers, and individual isomers are all included within the scope of the present disclosure.

The compounds of the present disclosure may be in the form of a particular geometric isomer or stereoisomer. All such compounds are contemplated herein, including cis and trans isomers, (-)- and (+)-enantiomers, (R)- and (S)-enantiomers, diastereoisomers, (D)-isomers, (L)-isomers, and racemic mixtures and other mixtures thereof, such as enantiomerically or diastereoisomerically enriched mixtures, all of which fall within the scope of the present disclosure. Additional asymmetric carbon atoms may be present in substituents such as alkyl. All these isomers and mixtures thereof are encompassed within the scope of the present disclosure.

The term "pharmaceutically acceptable carrier" refers to any preparation or carrier medium that is capable of delivering an effective amount of the active substance of the present disclosure, does not interfere with the biological activity of the active substance, and has no toxic or side effects in a host or a patient; representative carriers include, but are not limited to: binders, fillers, lubricants, disintegrants, wetting agents, dispersing agents, solubilizers, suspending agents, etc.

The "2θ" described in the present disclosure refers to the diffraction angle; the 2θ of each characteristic peak has a margin of error of ±0.20, including the case where a number having more than 1 decimal place is rounded.

In the present disclosure, there is a certain degree of error in the determination of the molar ratio of the compound of formula I to an acid molecule. Generally, ±10% falls within a reasonable margin of error; for example, the error may be ±9%, ±8%, ±7%, ±6%, ±5%, ±4%, ±3%, ±2%, or ±1%, preferably ±5%.

In the present disclosure, a numerical value described with "about" has the aforementioned reasonable margin of error.

The present disclosure is intended to include all isotopes of atoms present in the compounds of the present disclosure. Isotopes include those atoms having the same atomic number but different mass numbers. As a general and non-limiting example, isotopes of hydrogen include deuterium and tritium. Isotopes of carbon include ¹³C and ¹⁴C. Isotopically labeled compounds of the present disclosure can generally be prepared by conventional techniques known to those skilled in the art or by methods similar to those described herein using an appropriate isotopically labeled reagent in place of the non-labeled reagent otherwise used.

### BRIEF DESCRIPTION OF THE DRAWINGS

FIGs. 1-1, 1-2, and 1-3 show an XRPD pattern, TGA/DSC thermograms, and a ¹H NMR spectrum of the crystal form A of the compound of formula I, respectively.
FIGs. 2-1, 2-2, and 2-3 show an XRPD pattern, a ¹H NMR spectrum, and TGA/DSC thermograms of the crystal form A of the fumarate of the compound of formula I, respectively.
FIG. 2-4 shows an XRPD pattern of the crystal form B of the fumarate of the compound of formula I.
FIGs. 3-1, 3-2, and 3-3 show an XRPD pattern, a ¹H NMR spectrum, and TGA/DSC thermograms of the crystal form A of the L-malate of the compound of formula I, respectively.
FIG. 4-1 shows an XRPD pattern overlay of the crystal forms A/B/C of the hydrochloride of the compound of formula I.
FIGs. 4-2 and 4-3 show a TGA thermogram and a DSC thermogram of the crystal form A of the hydrochloride of the compound of formula I, respectively.
FIGs. 4-4 and 4-5 show a ¹H NMR spectrum and a TGA thermogram of the crystal form B of the hydrochloride of the compound of formula I, respectively.
FIG. 4-6 shows TGA/DSC thermograms of the crystal form C of the hydrochloride of the compound of formula I.
FIG. 5-1 shows an XRPD pattern overlay of the crystal forms A/B of the methanesulfonate of the compound of formula I.
FIGs. 5-2 and 5-3 show ¹H NMR spectra of the crystal forms A and B of the methanesulfonate of the compound of formula I, respectively.
FIGs. 6-1, 6-2, and 6-3 show an XRPD pattern, a ¹H NMR spectrum, and a TGA thermogram of the crystal form A of the benzenesulfonate of the compound of formula I, respectively.

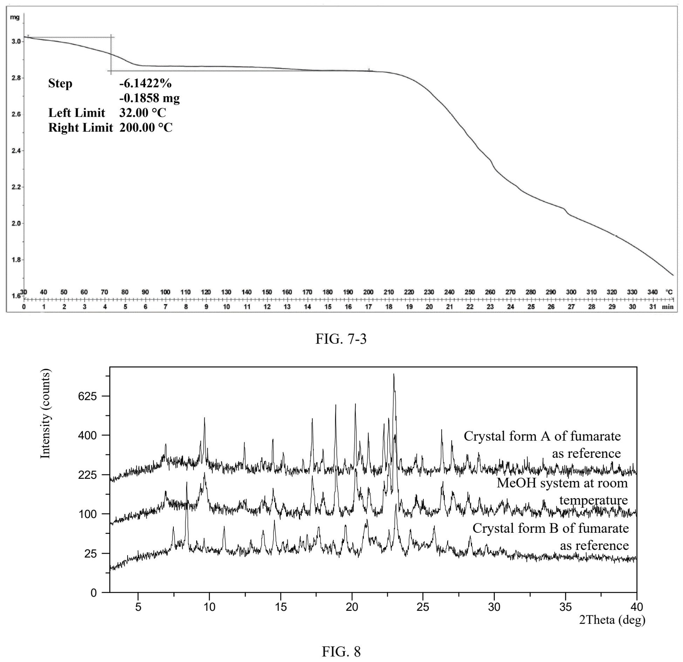

FIGs. 7-1, 7-2, and 7-3 show an XRPD pattern, a ¹H NMR spectrum, and a TGA thermogram of the crystal form A of the gentisate of the compound of formula I, respectively.
FIG. 8 shows an XRPD pattern comparison in a suspension competition test of the crystal forms A and B of the fumarate of the compound of formula I.

### DETAILED DESCRIPTION

The present disclosure is further illustrated below with reference to specific examples and test examples, but the scope of the present disclosure is not limited in any way.

Test conditions for the instruments used in the experiments:

### X-Ray powder diffraction (XRPD)

X-ray powder diffraction data for samples were collected under ambient conditions using a Bruker D2 X-ray powder diffractometer. A sample (about 2 mg) was taken, spread on a high-purity silicon sample stage, flattened using a glass slide with a weighing paper in between, and loaded for analysis. The X-ray tube used a Cu target (Kα), and the Kα2/Kα1 intensity ratio was 0.50 (1.54439 Å/1.5406 Å). The X-ray emitter had a power of 300 W, a voltage of 30 kV, and a current of 10 mA. The divergence slit was 0.6 mm, and the Soller slit was 4.0°. The step rate was 0.15 s/step, the step size was 0.02° (2θ), and the total number of steps was 1837.

### Thermogravimetric analyzer (TGA)

Thermogravimetric data for samples were collected using a TA Discovery-series thermogravimetric analyzer (TGA550). A sample (several milligrams) was taken, placed in a Tzero aluminum pan (the sample was automatically weighed by the instrument in the test), and heated from room temperature to the target temperature in a N₂ atmosphere, with a N₂ flow rate of 60 mL/min and a ramp rate of 10 °C/min.

### Differential scanning calorimeter (DSC)

Thermal data for samples were collected using a TA Discovery-series differential scanning calorimeter (DSC2500). A sample (several milligrams) was weighed into a Tzero aluminum pan, and the pan was sealed with a Tzero hermetic lid. The sample was heated to the target temperature (before the decomposition temperature) in a N₂ atmosphere, with a N₂ flow rate of 50 mL/min and a ramp rate of 10 °C/min.

### Proton nuclear magnetic resonance spectroscopy (¹H NMR)

Samples were dissolved in DMSO-*d₆* to form solutions with a concentration of about 2-10 mg/mL, and proton nuclear magnetic resonance spectroscopic data for the samples were collected using a Bruker AVANCE NEO 400 MHZ.

### High performance liquid chromatography (HPLC)

The purity of the samples was determined using an Agilent 1260 high performance liquid chromatograph (equipped with a DAD detector) with a Zorbax Eclipse XDB-C18, 4.6 × 250 mm, 5 µm column.

### Example 1. Synthesis and Characterization of Compound of Formula I

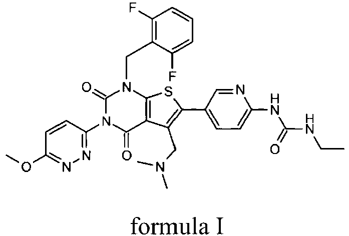

### Synthesis scheme:

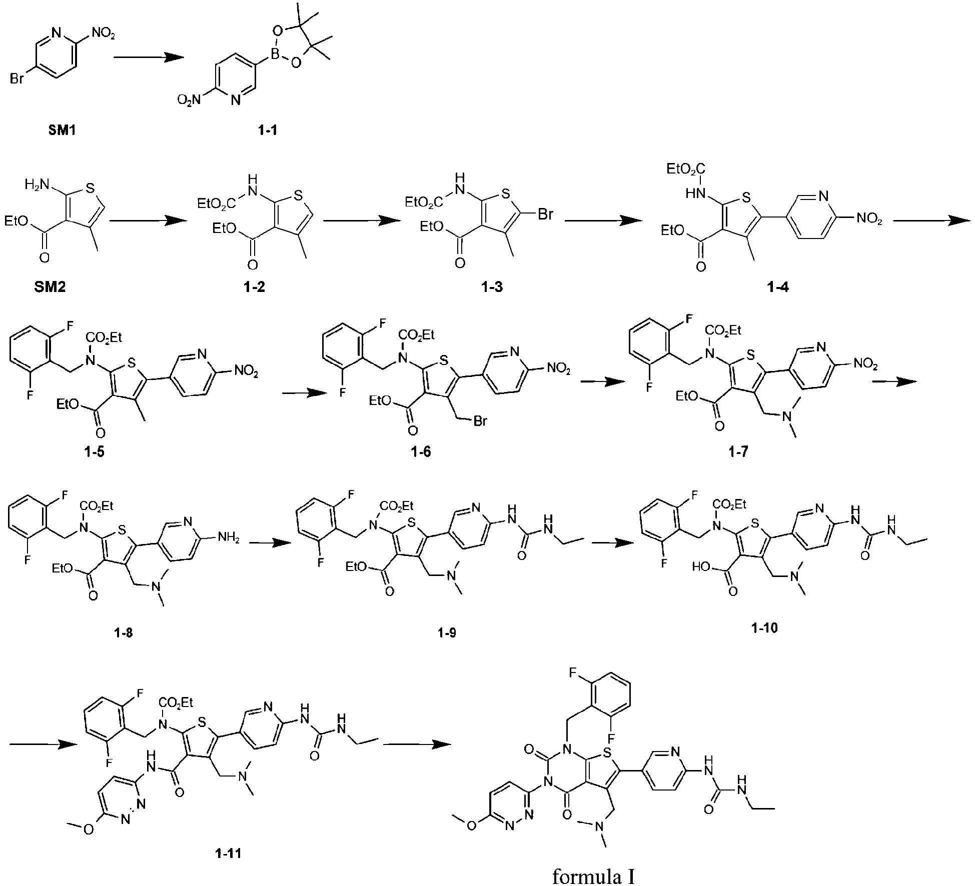

### Synthesis of compound 1-1:

Bis(pinacolato)diboron (75.1 g, 296 mmol, 1.20 eq) and Pd(dppf)Cl₂ (7.21 g, 9.85 mmol, 0.04 eq) were added to a solution of SM1 (50.0 g, 246 mmol, 1.00 eq) and AcOK (120.87 g, 1.23 mol, 5.00 eq) in dioxane (600 mL) in a N₂ atmosphere. The mixture was stirred at 100 °C for 1.5 h. LCMS showed that SM1 had been consumed, and a main peak had been detected. The reaction mixture was filtered through diatomaceous earth. The filtrate was concentrated under vacuum to give a residue. The residue was purified by silica gel column chromatography (petroleum ether/ethyl acetate = 75/25) and slurried with petroleum ether/ethyl acetate (5/1, 420 mL) at 25 °C for 2 h to give compound 1-1 (20 g, 77.5 mmol, yield: 72.0%, purity: 96.9%) as a white solid. MS m/z (ESI): 169[M+H]⁺; ¹H NMR (400 MHz CDCl₃), δppm 8.94 (s, 1 H), 8.38 - 8.40 (m, 1 H), 8.21 (d, *J* = 8.00 Hz, 1 H), 1.37 (s, 12 H).

### Step 1: synthesis of compound 1-2:

Ethyl chloroformate (58.9 g, 542 mmol, 51.7 mL, 5.03 eq) was added to a solution of SM2 (20.0 g, 108 mmol, 1.00 eq) in toluene (200 mL). The mixture was stirred at 110 °C for 16 h. LCMS showed that SM3 had been consumed, and a main peak had been detected. The mixture was concentrated under reduced pressure to give a residue, and the residue was directly used in the next step without purification. Compound 1-2 (27.6 g, 97.7 mmol, yield: 90.5%, purity: 91.1%) was obtained as a grey solid. MS m/z (ESI): 258[M+H]⁺.

### Step 2: synthesis of compound 1-3:

NBS (20.6 g, 115.9 mmol, 1.10 eq) was added to a solution of compound 2-2 (27.1 g, 105.32 mmol, 1 eq) in CHCl₃ (220 mL) at 0 °C. The mixture was stirred at 0 °C for 2 h. LCMS showed that compound 2-2 had been consumed, and a main peak had been detected. The mixture was concentrated under reduced pressure to give a residue. The residue was purified by column chromatography (SiO2, petroleum ether/ethyl acetate = 94/6) to give compound 1-3 (36.2 g, 93.0 mmol, yield: 71.1%, purity: 86.293%) as a white solid. MS m/z (ESI): 336,338[M+H]⁺; ¹H NMR (400 MHz DMSO-d6), δ ppm 4.20 - 4.29 (m, 4 H), 2.22 (s, 3H), 1.24 - 1.33 (m, 6H).

### Step 3: synthesis of compound 1-4:

Compound 1-1 (33.92 g, 135.63 mmol, 2.4 eq), K₂CO₃ (15.62 g, 113.03 mmol, 2 eq), and Pd(dppf)Cl₂ (4.14 g, 5.65 mmol, 0.1 eq) were added to a solution of compound 1-3 (19 g, 56.51 mmol, 1 eq) in dioxane (180 mL) and H₂O (18 mL). The mixture was stirred at 80 °C for 18 h. LCMS showed that compound 1-3 had been consumed, and many new peaks had been formed. The mixture was poured into water (100 mL), and the resulting mixture was extracted with ethyl acetate (100 mL × 3). The organic layers were combined, washed with brine (200 mL), dried over sodium sulfate, filtered, and concentrated under reduced pressure to give a residue. The residue was purified by column chromatography (SiO₂, petroleum ether/dichloromethane = 30/70, Rf = 0.26) to give compound 1-4 (9.48 g, 22.80 mmol, yield: 19.22%, purity: 91.226%) as a yellow solid. MS m/z (ESI): 380[M+H]⁺.

### Step 4: synthesis of compound 1-5:

2,6-Difluorobenzyl chloride (3.09 g, 19.0 mmol, 1.20 eq) was added to a solution of compound 1-4 (6.00 g, 15.8 mmol, 1.00 eq), KI (2.89 g, 17.4 mmol, 1.10 eq), and K₂CO₃ (2.40 g, 17.4 mmol, 1.10 eq) in DMF (50 mL), and the mixture was then stirred at 25 °C for 6 h. LCMS showed that compound 1-4 had been consumed, and a major peak had been formed. The mixture was poured into water (100 mL), and the resulting mixture was extracted with ethyl acetate (100 mL × 3). The extract was washed with brine (200 mL × 1), dried over anhydrous Na₂SO₄, and concentrated under reduced pressure to remove the solvent. The residue was purified by column chromatography (SiO₂, petroleum ether/ethyl acetate = 80/20) to give compound 1-5 (7.26 g, 14.3 mmol, yield: 90.6%, purity: 99.8%) as a yellow oil. MS m/z (ESI): 506[M+H]⁺; ¹H NMR (400 MHz CDCl₃), δ ppm 8.60 (d, *J =* 1.75 Hz, 1 H), 8.30 (d, *J =* 8.38 Hz, 1 H), 7.99 - 8.01 (m, 1 H), 7.24 - 7.30 (m, 1 H), 6.82 - 6.88 (m, 2 H), 4.97 (s, 2 H), 4.08 - 4.27 (m, 4 H), 2.40 (s, 3 H), 1.30 (m, 3 H), 1.18 - 1.25 (m, 3 H).

### Step 5: synthesis of compound 1-6:

NBS (3.07 g, 17.2 mmol, 1.20 eq) and AIBN (235.84 mg, 1.44 mmol, 0.1 eq) were added to a solution of compound 1-5 (7.26 g, 14.4 mmol, 1.00 eq) in ethyl acetate (50 mL), and the mixture was then stirred at 80 °C for 16 h. NBS (3.07 g, 17.2 mmol, 1.20 eq) and AIBN (236 mg, 1.44 mmol, 0.10 eq) were added, and the mixture was stirred at 80 °C for 4 h. NBS (3.07 g, 17.2 mmol, 1.20 eq) and AIBN (236 mg, 1.44 mmol, 0.10 eq) were added, and the mixture was stirred at 80 °C for 16 h. LCMS showed that compound 1-5 had been consumed, and a major peak had been formed. The mixture was diluted with ethyl acetate (100 mL) and washed with brine (50 mL × 2). The aqueous layer was extracted with ethyl acetate (50 mL). The organic layer was dried over Na₂SO₄ and concentrated under reduced pressure to remove the solvent. The residue was directly used in the next step without purification. Compound 1-6 (13 g, crude product) was obtained as a brown oil. MS m/z (ESI): 584,586[M+H]⁺.

### Step 6: synthesis of compound 1-7:

DIEA (7.19 g, 55.6 mmol, 9.69 mL, 2.50 eq) was added to a solution of compound 1-6 (13.0 g, 22.3 mmol, 1.00 eq) in DMF (100 mL), and Me₂NH.HCl (2.39 g, 29.4 mmol, 1.32 eq) was then added in portions. The mixture was stirred at 25 °C for 5 h. LCMS showed that compound 1-6 had been consumed, and a major peak had been formed. The mixture was poured into water (100 mL), and the resulting mixture was extracted with ethyl acetate (150 mL × 3). The extract was washed with brine (100 mL × 2), dried over Na₂SO₄, and concentrated under reduced pressure to remove the solvent. The residue was purified by silica gel column chromatography (petroleum ether/ethyl acetate = 60/40) to give compound 1-7 (4.10 g, 6.30 mmol, yield: 28.3%, purity: 84.3%) as a yellow oil. MS m/z (ESI): 549[M+H]⁺; ¹H NMR (400 MHz CDCl₃), δ ppm 8.76 (d, *J =* 1.38 Hz, 1 H), 8.24 - 8.30 (m, 2 H), 7.22 - 7.28 (m, 1 H), 6.81 - 6.86 (m, 2 H), 5.00 (s, 2 H), 4.18 - 4.26 (m, 4 H), 3.53 (s, 2 H), 2.10 (s, 6 H), 1.18 - 1.32 (m, 7 H).

### Step 7: synthesis of compound 1-8:

Pd/C (0.6 g, 1.13 mmol, purity: 10%, 0.20 eq) was added to a solution of compound 1-7 (3.10 g, 5.65 mmol, 1.00 eq) in EtOH (30 mL) in a N₂ atmosphere, and the mixture was degassed under vacuum and purged three times with H2. The mixture was stirred in a H₂ atmosphere (20 psi) at 25 °C for 34 h. LCMS showed that compound 1-7 had been consumed, and a major peak had been formed. The reaction system was filtered through diatomaceous earth. The filter cake was washed with 10 mL of ethanol, and the filtrate was concentrated under reduced pressure to remove the solvent. Tetrahydrofuran (THF, 30 mL) was added to the residue, and the mixture was distilled to remove the solvent; this process was repeated three times. The residue was purified by silica gel column chromatography (dichloromethane/methanol = 92/8) to give compound 1-8 (1.80 g, 2.83 mmol, yield: 48.8%, purity: 81.4%) as a yellow oil. MS m/z (ESI): 519[M+H]⁺; ¹H NMR (400 MHz CDCl₃), δ ppm 8.04 (d, *J* = 2.13 Hz, 1 H), 7.47 (dd, *J* = 8.44, 2.31 Hz, 1 H), 7.20 - 7.27 (m, 1 H), 6.80 - 6.86 (m, 2 H), 6.52 (d, *J =* 8.51 Hz, 1 H), 4.98 (s, 2 H), 4.70 (s, 2 H), 4.16 - 4.26 (m, 4 H), 3.54 - 3.70 (m, 2 H), 2.12 (s, 6 H), 1.16 - 1.32 (m, 6 H).

### Step 8: synthesis of compound 1-9:

C₂H₅NCO (822 mg, 11.6 mmol, 916 µL, 10.0 eq) was added to a solution of compound 1-8 (1.20 g, 1.16 mmol, 1.00 eq) in THF (2 mL), and the mixture was stirred at 50 °C for 12 h. LCMS showed that compound 1-8 had been consumed, and a major peak had been formed. The reaction system was concentrated under reduced pressure to remove the solvent to give a residue. The residue was purified by silica gel column chromatography (dichloromethane/methanol = 10/1) to give compound 1-9 (1.01 g, crude product) as a yellow oil. MS m/z (ESI): 590[M+H]⁺; ¹H NMR (400 MHz CDCl₃), δ ppm 9.17 (s, 1 H), 8.80 (s, 1 H), 8.19 (d, *J =* 2.00 Hz, 1 H), 7.66 (dd, *J =* 8.50, 2.25 Hz, 1 H), 7.22 - 7.29 (m, 2 H), 6.93 (d, *J =* 8.38 Hz, 1 H), 6.82 - 6.86 (m, 2 H), 5.00 (s, 2 H), 4.18 - 4.29 (m, 4 H), 3.39 - 3.46 (m, 2 H), 3.15 - 3.34 (m, 2 H), 2.17 (s, 6 H), 1.32 (t, *J =* 7.13 Hz, 3 H), 1.23 - 1.27 (m, 3 H), 1.15 - 1.19 (m, 3 H).

### Step 9: synthesis of compound 1-10:

Compound 1-9 (1.01 g, 1.72 mmol, 1.00 eq) in EtOH (10 mL) was added to a NaOH (2 M, 4.29 mL, 5.00 eq) solution, and the mixture was stirred at 60 °C for 2 h. LCMS showed that compound 1-9 had been consumed, and a major peak had been formed. The pH of the reaction solution was adjusted to 5-7 with HCl (1 mol/L, about 8 mL) at 0 °C. The mixture was concentrated under reduced pressure and lyophilized to remove the solvent to give a residue. The residue was purified by silica gel column chromatography (dichloromethane/methanol = 87/13) to give compound 1-10 (798 mg, 1.20 mmol, yield: 70.1%, purity: 84.7%) as a yellow solid. MS m/z (ESI): 562[M+H]⁺; ¹H NMR (400 MHz CDCl₃), δ ppm 9.43 (s, 1 H), 7.97 - 8.04 (m, 2 H), 7.51 - 7.60 (m, 2 H), 7.36 - 7.43 (m, 1 H), 7.04 (t, *J =* 8.00 Hz, 2 H), 4.89 (s, 2 H), 4.01 - 4.05 (m, 2 H), 3.11 - 3.21 (m, 3 H), 2.94 - 3.01 (m, 1 H), 2.44 (s, 6 H), 1.06 - 1.10 (m, 3 H), 0.94 - 1.04 (m, 3 H).

### Step 10: synthesis of compound 1-11:

Compound 1-10 (220 mg, 356 µmol, 1.00 eq) and 3-amino-6-methoxypyridazine (80.2 mg, 641 µmol, 1.80 eq) were dissolved in DMF (6 mL), and DIEA (460 mg, 3.56 mmol, 620 µL, 10.00 eq) and T₃P (680 mg, 1.07 mmol, 635 µL, purity: 50%, 3.00 eq) were added. The mixture was stirred at 25 °C for 17 h. Compound 12a (89.1 mg, 712 µmol, 2.00 eq), DIEA (460 mg, 3.56 mmol, 620 µL, 10.00 eq), and T₃P (680 mg, 1.07 mmol, 635 µL, purity: 50%, 3.00 eq) were added, and the mixture was stirred at 25 °C for 1 h. Compound 12a (89.1 mg, 712 µmol, 2.00 eq), DIEA (460 mg, 3.56 mmol, 620 µL, 10 eq), and T₃P (680 mg, 1.07 mmol, 635 µL, purity: 50%, 3.00 eq) were additionally added, and the mixture was stirred at 25 °C for another hour. LCMS showed that compound 1-10 had been consumed, and a major peak had been formed. The mixture was poured into water (12 mL), and the resulting mixture was extracted with ethyl acetate (3 × 10.0 mL). The organic phase was washed with brine (3 × 30.0 mL), dried over anhydrous sodium sulfate, filtered, and concentrated to give a residue. Compound 1-11 (270 mg, crude product) was obtained as a yellow oil. MS m/z (ESI): 669[M+H]⁺.

### Step 11: synthesis of the compound of formula I:

MeONa (218 mg, 4.04 mmol, 10.00 eq) was added to a solution of compound 1-11 (270 mg, 404 µmol, 1.00 eq) in MeOH (5 mL) at 0 °C, and the mixture was stirred at 25 °C for 14 h. LCMS showed that compound 1-11 had been consumed, and many peaks had been formed. The reaction was quenched with an aq. NH₄Cl solution (13 mL). Then the mixture was extracted with ethyl acetate (3 × 10.0 mL) and chloromethane (3 × 10.0 mL). The organic phase was washed with brine (50.0 mL), dried over anhydrous sodium sulfate, filtered, and concentrated to give a residue. The residue was purified by silica gel column chromatography (dichloromethane/methanol = 10/1) and column chromatography (Welch xltimate C18 150 × 25 mm × 5 µm; mobile phase: [water (NH4HCO3)-acetonitrile (ACN)]; B%: 10%-85%, 12 min) to give the compound of formula I (25.0 mg, 40.06 µmol, yield: 9.92%, purity: 99.8%) as a white solid. MS m/z (ESI): 562[M+H]⁺; ¹H NMR (400 MHz CD₃OD), δ ppm 8.31 (d, *J =* 2.13 Hz, 1 H), 7.82 (dd, *J* =8.63, 2.38 Hz, 1 H), 7.64 (d, *J* = 9.13 Hz, 1 H), 7.32 - 7.49 (m, 2 H), 7.19 (d, *J* = 8.63 Hz, 1 H), 6.93 - 7.07 (m, 2 H), 5.26 - 5.49 (m, 2 H), 4.14 (s, 3 H), 3.29 - 3.36 (m, 4 H), 2.07 (s, 6 H), 1.19 (t, *J* =7.25 Hz, 3 H). ¹⁹F NMR: (400 MHz CDCl₃) δ -114.827 ppm.

### Characterization of the compound of formula I:

As can be seen from XRPD (FIG. 1-1), the compound of formula I was a crystal with relatively high crystallinity, designated crystal form A. Its characteristic peaks are shown in Table 1. Its purity was 99.54 area%. The TGA/DSC results (FIG. 1-2) show that the sample had a single melting peak at 260.3 °C (starting temperature) and a weight loss of 0.9% before 220 °C (0.5 H₂O corresponds to 1.4 wt%). The quantitative ¹H NMR spectrum (FIG. 1-3, CDCl₃) was consistent with the structure of the compound of formula I. The average weight content was 97.6%, and the residual DMSO solvent content was about 0.7 wt% (the molar ratio of DMSO to the compound of formula I was 0.06). Considering that the relatively low weight loss in TGA should be a small amount of residual DMSO, the crystal form A was an anhydrous crystal form.

**Table 1. Analytical data of the XRPD pattern of the crystal form A of the compound of formula I**

| No. | 2θ±0.2 (°) | Relative intensity (%) | No. | 2θ±0.2 (°) | Relative intensity (%) |
|---|---|---|---|---|---|
| 1 | 7.2390 | 27.89 | 15 | 21.5519 | 6.00 |
| 2 | 8.7232 | 20.55 | 16 | 22.1387 | 43.95 |
| 3 | 10.5072 | 48.34 | 17 | 23.4047 | 29.10 |
| 4 | 10.9392 | 12.74 | 18 | 23.9560 | 3.58 |
| 5 | 11.8203 | 6.43 | 19 | 24.2983 | 6.44 |
| 6 | 12.4670 | 100 | 20 | 25.1171 | 9.88 |
| 7 | 12.9697 | 4.85 | 21 | 25.8572 | 8.52 |
| 8 | 14.9748 | 11.37 | 22 | 26.6540 | 7.48 |
| 9 | 16.0143 | 3.88 | 23 | 27.3319 | 18.13 |
| 10 | 17.6527 | 20.23 | 24 | 29.2089 | 2.54 |
| 11 | 18.6162 | 69.93 | 25 | 29.5722 | 4.78 |
| 12 | 19.6161 | 58.54 | 26 | 31.0191 | 7.87 |
| 13 | 20.4808 | 9.74 | 27 | 32.1255 | 2.73 |
| 14 | 21.1511 | 8.54 | 28 | 36.0622 | 2.58 |

### Example 2. Fumarate of Compound of Formula I

### 2.1. Preparation and characterization of crystal form A of fumarate

The compound of formula I prepared in Example 1 (25 mg) and fumaric acid (in an equimolar ratio) were weighed out, and 0.5-1.0 mL of methanol:dichloromethane (1:1, v/v) was added. After the mixture was magnetically stirred at room temperature for 2.5 days, a solid was centrifugally separated and dried under vacuum at 40 °C for 3 h to give the crystal form A of the fumarate of the compound of formula I, with a chemical purity of 99.78 area%. Its XRPD pattern (FIG. 2-1) shows relatively high crystallinity, and its characteristic peaks are shown in Table 2. The ¹H NMR (FIG. 2-2, DMSO-d6) results indicate that the molar ratio of fumaric acid to the compound of formula I in the sample was 0.9, and no additional solvent signals were observed. The TGA/DSC results (FIG. 2-3) show that the sample had a weight loss of 0.2527% at 200 °C and a sharp melting peak at 230.37 °C (peak value). The crystal form A of the fumarate was an anhydrous crystal form of a monofumarate.

**Table 2. Analytical data of the XRPD pattern of the crystal form A of the fumarate of the compound of formula I**

| No. | 2θ±0.2 (°) | Relative intensity (%) | No. | 2θ±0.2 (°) | Relative intensity (%) |
|---|---|---|---|---|---|
| 1 | 6.9355 | 17.43 | 14 | 20.5609 | 20.12 |
| 2 | 9.3706 | 13.25 | 15 | 21.1589 | 28.33 |
| 3 | 9.6549 | 42.59 | 16 | 22.2396 | 38.51 |
| 4 | 9.8505 | 8.76 | 17 | 22.5989 | 43.78 |
| 5 | 12.4723 | 19.53 | 18 | 22.9352 | 100.00 |
| 6 | 14.4518 | 25.18 | 19 | 23.4150 | 6.57 |
| 7 | 15.1922 | 13.97 | 20 | 24.4730 | 8.05 |
| 8 | 16.6040 | 6.60 | 21 | 24.9459 | 10.43 |
| 9 | 17.2206 | 45.51 | 22 | 26.2998 | 32.45 |
| 10 | 17.9747 | 16.33 | 23 | 27.0370 | 18.89 |
| 11 | 18.8612 | 62.26 | 24 | 28.1337 | 10.64 |
| 12 | 19.5004 | 7.94 | 25 | 28.9063 | 13.32 |
| 13 | 20.2529 | 59.83 | | | |

After standing for 30 days under a high temperature (60 °C) condition, standing for 30 days under a high humidity (25 °C/92.5% RH) condition, standing for 10 days under an illumination condition (meeting 1.2 × 106 lux·hr-visible light and 200 W-hr/m2-near ultraviolet), standing for 3 months under an acceleration condition (40 °C ± 2 °C/75% RH ± 5% RH), or standing for 3 months under a long-term condition (30 °C ± 2 °C/65% RH ± 5% RH), the crystal form A of the fumarate of the compound of formula I showed no changes in content or related substances and remained unchanged, compared to day 0, indicating that the crystal form A of the fumarate had relatively good physical and chemical stability.

### 2.2. Preparation and characterization of crystal form B of fumarate

The crystal form A of the fumarate (20 mg) was weighed out, and an EtOH anti-solvent was added to an EtOH/H₂O (1:1) system. After 15 days of volatilization in an open flask at room temperature (25 °C), a small amount of solid precipitated out on the wall of the flask (the crystal form B of the fumarate of the compound of formula I), and a large amount of solid precipitated out at the bottom of the flask (the crystal form A of the fumarate of the compound of formula I). The XRPD pattern of the crystal form B of the fumarate is shown in FIG. 2-4, and its characteristic peaks are shown in Table 3.

**Table 3. Analytical data of the XRPD pattern of the crystal form B of the fumarate of the compound of formula I**

| No. | 2θ±0.2 (°) | Relative intensity (%) | No. | 2θ±0.2 (°) | Relative intensity (%) |
|---|---|---|---|---|---|
| 1 | 7.4623 | 23.00 | 25 | 18.6842 | 10.15 |
| 2 | 8.4044 | 100.00 | 26 | 19.5247 | 28.95 |
| 3 | 9.1115 | 5.76 | 27 | 20.9439 | 23.13 |
| 4 | 9.6295 | 8.70 | 28 | 21.1012 | 27.79 |
| 5 | 11.0463 | 26.37 | 29 | 21.4357 | 9.77 |
| 6 | 12.9182 | 8.80 | 30 | 21.6687 | 9.67 |
| 7 | 13.7771 | 20.80 | 31 | 22.6092 | 17.33 |
| 8 | 14.5639 | 32.58 | 32 | 23.0754 | 54.86 |
| 9 | 15.1602 | 7.91 | 33 | 24.1136 | 21.19 |
| 10 | 15.4508 | 6.07 | 34 | 25.7680 | 22.00 |
| 11 | 16.4396 | 5.91 | 35 | 26.6979 | 8.87 |
| 12 | 16.8553 | 14.53 | 36 | 28.2978 | 16.22 |
| 13 | 17.6799 | 23.69 | 37 | 29.4248 | 7.82 |

### Example 3. Preparation and Characterization of Crystal Form A of L-Malate of Compound of Formula I

The compound of formula I (25 mg) and L-malic acid (in an equimolar ratio) were weighed out, and 0.5-1.0 mL of EtOAc (ethyl acetate) was added. After the mixture was magnetically stirred at room temperature for 2.5 days, cycles of heating and cooling (50 °C-20 °C-50 °C-20 °C, heating and cooling rate: 10 °C/h) were performed, and a solid was centrifugally separated and dried under vacuum at 40 °C for 3 h to give the crystal form A of the L-malate of the compound of formula I, with a chemical purity of 99.63 area%. The XRPD results (FIG. 3-1) indicate that the sample had relatively high crystallinity, and its characteristic peak positions are shown in Table 4. The ¹H NMR (FIG. 3-2, DMSO-d6) results indicate that the molar ratio of L-malic acid to the compound of formula I in the sample was 1.0, and the residual EtOAc solvent content was about 0.7 wt% (EtOAc:the compound of formula I = 0.06). The TGA/DSC results (FIG. 3-3) show that the sample had a weight loss of 0.1214% at 150 °C and a sharp melting peak at 183.63 °C (peak value). The crystal form A of the L-malate was an anhydrous crystal form of a mono-L-malate.

**Table 4. Analytical data of the XRPD pattern of the crystal form A of the L-malate of the compound of formula I**

| No. | 2θ±0.2 (°) | Relative intensity (%) | No. | 2θ±0.2 (°) | Relative intensity (%) |
|---|---|---|---|---|---|
| 1 | 6.8934 | 4.71 | 25 | 19.5753 | 15.07 |
| 2 | 9.2046 | 30.31 | 26 | 20.1444 | 56.21 |
| 3 | 9.6751 | 53.00 | 27 | 20.6407 | 42.20 |
| 4 | 10.3958 | 6.61 | 28 | 21.1466 | 22.85 |
| 5 | 12.4714 | 22.69 | 29 | 22.2793 | 69.89 |
| 6 | 13.5073 | 25.05 | 30 | 22.8785 | 100.00 |
| 7 | 14.4312 | 21.74 | 31 | 23.1632 | 60.28 |
| 8 | 15.2262 | 6.03 | 32 | 24.2230 | 9.28 |
| 9 | 16.4629 | 9.63 | 33 | 26.2217 | 21.59 |
| 10 | 17.1769 | 69.13 | 34 | 27.0313 | 20.83 |
| 11 | 17.5934 | 10.79 | 35 | 27.6596 | 7.49 |
| 12 | 18.0032 | 24.67 | 36 | 28.0101 | 8.10 |
| 13 | 18.6031 | 25.05 | 37 | 28.9051 | 5.20 |
| 14 | 18.8495 | 41.83 | 38 | | |

### Example 4. Hydrochloride of Compound of Formula I

### 4.1. Preparation and characterization of crystal form A of hydrochloride

The compound of formula I (25 mg) and hydrochloric acid (in an equimolar ratio) were weighed out, and 0.5-1.0 mL of acetone was added. After the mixture was magnetically stirred at room temperature for 2.5 days, a solid was centrifugally separated and dried under vacuum at 40 °C for 3 h to give the crystal form A of the hydrochloride of the compound of formula I, with a chemical purity of 99.15 area%. The IC results show that the content of Cl⁻ was 5.7%, the content of the compound of formula I was 90.3%, and the molar ratio of hydrochloric acid to the compound of formula I was 1.1. The XRPD results (FIG. 4-1) indicate medium crystallinity, and the characteristic peaks are shown in Table 5. The TGA results (FIG. 4-2) indicate that the sample had a weight loss of 6.4% before 103 °C, which corresponds to about 2.5 H₂O molecules. The DSC results (FIG. 4-3) show that the sample had a broad endothermic peak (70.62 °C, peak value) before melting (206.86 °C, peak value), presumably due to dehydration. The crystal form A of the hydrochloride should be a hydrate of monohydrochloride.

**Table 5. Analytical data of the XRPD pattern of the crystal form A of the hydrochloride of the compound of formula I**

| No. | 2θ±0.2 (°) | Relative intensity (%) | No. | 2θ±0.2 (°) | Relative intensity (%) |
|---|---|---|---|---|---|
| 1 | 9.1252 | 99.72 | 7 | 17.5138 | 51.91 |
| 2 | 9.6045 | 27.25 | 8 | 20.9172 | 47.49 |
| 3 | 10.4351 | 78.40 | 9 | 21.6060 | 61.91 |
| 4 | 11.2797 | 100.00 | 10 | 24.5332 | 21.80 |
| 5 | 12.9108 | 44.35 | 11 | 26.0138 | 27.29 |
| 6 | 17.0513 | 38.63 | | | |

### 4.2. Preparation and characterization of crystal form B of hydrochloride

The compound of formula I (25 mg) and hydrochloric acid (in an equimolar ratio) were weighed out, and 0.5-1.0 mL of ACN was added. After the mixture was magnetically stirred at room temperature for 2.5 days, a solid was centrifugally separated and dried under vacuum at 40 °C for 3 h to give the crystal form B of the hydrochloride of the compound of formula I, with a chemical purity of 99.67 area%. The IC results show that the content of Cl⁻ was 5.2%, the content of the compound of formula I was 91.6%, and the molar ratio of hydrochloric acid to the compound of formula I was 1.0. The XRPD results (FIG. 4-1) indicate medium crystallinity, and the characteristic peaks are shown in Table 6. According to the ¹H NMR (FIG. 4-4, DMSO-*d₆*) spectrum, the residual ACN solvent content was about 3.9 wt% (ACN:the compound of formula I = 0.7). The TGA results (FIG. 4-5) indicate that the sample had a weight loss of 1.4% before 100 °C, which corresponds to about 0.2 ACN molecules. The crystal form B of the hydrochloride of the compound of formula I was an ACN solvate of a monohydrochloride.

**Table 6. Analytical data of the XRPD pattern of the crystal form B of the hydrochloride of the compound of formula I**

| No. | 2θ±0.2 (°) | Relative intensity (%) | No. | 2θ±0.2 (°) | Relative intensity (%) |
|---|---|---|---|---|---|
| 1 | 5.9104 | 4.10 | 25 | 21.7332 | 16.99 |
| 2 | 9.4060 | 12.80 | 26 | 22.2546 | 8.98 |
| 3 | 10.0584 | 16.64 | 27 | 22.6955 | 16.08 |
| 4 | 12.0366 | 26.67 | 28 | 23.6400 | 23.96 |
| 5 | 13.2712 | 17.35 | 29 | 23.8466 | 33.33 |
| 6 | 14.9457 | 44.61 | 30 | 24.9835 | 57.85 |
| 7 | 15.9074 | 3.83 | 31 | 25.2298 | 19.71 |
| 8 | 16.6271 | 10.61 | 32 | 25.6467 | 10.00 |
| 9 | 17.3666 | 100.00 | 33 | 27.2387 | 22.38 |
| 10 | 17.5949 | 24.72 | 34 | 28.3166 | 7.99 |
| 11 | 19.5860 | 6.75 | 35 | 28.7581 | 19.12 |
| 12 | 20.0533 | 10.56 | 36 | 30.2705 | 15.10 |
| 13 | 20.2917 | 9.35 | 37 | 35.0987 | 4.27 |

### 4.3. Preparation and characterization of crystal form C of hydrochloride

The compound of formula I (25 mg) and hydrochloric acid (in an equimolar ratio) were weighed out, and 0.5-1.0 mL of MeOH:DCM (1:1, v/v) was added. After the mixture was magnetically stirred at room temperature for 2.5 days, the mixture was stirred at 5 °C for 1 day and left to stand at -20 °C for 1 day. After MTBE (methyl tert-butyl ether) was added as an anti-solvent, a solid was centrifugally separated and dried under vacuum at 40 °C for 3 h to give the crystal form C of the hydrochloride of the compound of formula I, with a chemical purity of 99.02 area%. The IC results show that the content of Cl⁻ was 5.0%, the content of the compound of formula I was 86.6%, and the molar ratio of hydrochloric acid to the compound of formula I was 1.0. The XRPD results (FIG. 4-1) indicate medium crystallinity, and the characteristic peaks are shown in Table 7. The TGA/DSC results (FIG. 4-6) indicate that the sample had a weight loss of 4.167% before 120 °C, which corresponds to about 1.5 H₂O molecules, and a broad endothermic peak (82.07 °C, peak value) before melting (204.23 °C, peak value), presumably due to dehydration. The crystal form C of the hydrochloride of the compound of formula I was a hydrate of a monohydrochloride.

**Table 7. Analytical data of the XRPD pattern of the crystal form C of the hydrochloride of the compound of formula I**

| No. | 2θ±0.2 (°) | Relative intensity (%) | No. | 2θ±0.2 (°) | Relative intensity (%) |
|---|---|---|---|---|---|
| 1 | 8.3817 | 100.00 | 5 | 16.1577 | 34.35 |
| 2 | 9.1554 | 17.09 | 6 | 16.9678 | 21.96 |
| 3 | 10.6700 | 61.62 | 7 | 18.5478 | 19.60 |
| 4 | 11.3268 | 59.89 | 8 | 21.5630 | 35.28 |

### Example 5. Methanesulfonate of Compound of Formula I

### 5.1. Preparation and characterization of crystal form A of methanesulfonate

The compound of formula I (25 mg) and methanesulfonic acid (in an equimolar ratio) were weighed out, and 0.5-1.0 mL of ACN was added. After the mixture was magnetically stirred at room temperature for 2.5 days, a solid was centrifugally separated and dried under vacuum at 40 °C for 3 h to give the crystal form A of the methanesulfonate of the compound of formula I, with a chemical purity of 99.72 area%. The XRPD results are shown in FIG. 5-1, and the characteristic peaks are shown in Table 8. The ¹H NMR (FIG. 5-2, DMSO-*d₆*) results indicate that the molar ratio of methanesulfonic acid to the compound of formula I in the sample was 1.0, the residual ACN solvent content was about 3.0 wt% (ACN:the compound of formula I = 0.5), and the residual EtOAc solvent content (contamination resulting from vacuum drying) was about 1.7 wt% (EtOAc:the compound of formula I = 0.1).

**Table 8. Analytical data of the XRPD pattern of the crystal form A of the methanesulfonate of the compound of formula I**

| No. | 2θ±0.2 (°) | Relative intensity (%) | No. | 2θ±0.2 (°) | Relative intensity (%) |
|---|---|---|---|---|---|
| 1 | 6.6779 | 12.30 | 9 | 17.0799 | 16.49 |
| 2 | 7.2085 | 23.63 | 10 | 19.3276 | 6.85 |
| 3 | 9.9783 | 100.00 | 11 | 19.7556 | 14.58 |
| 4 | 10.5584 | 18.97 | 12 | 21.0763 | 12.77 |
| 5 | 12.8184 | 5.00 | 13 | 22.9116 | 6.53 |
| 6 | 15.3689 | 20.67 | 14 | 24.1325 | 5.88 |
| 7 | 15.7572 | 13.26 | 15 | 25.8708 | 10.65 |
| 8 | 16.2460 | 10.17 | | | |

### 5.2. Preparation and characterization of crystal form B of methanesulfonate

The compound of formula I (25 mg) and methanesulfonic acid (in an equimolar ratio) were weighed out, and 0.5-1.0 mL of acetone was added. After the mixture was magnetically stirred at room temperature for 2.5 days, cycles of heating and cooling (50 °C-20 °C-50 °C-20 °C, heating and cooling rate: 10 °C/h) were performed, and a solid was centrifugally separated and dried under vacuum at 40 °C for 3 h to give the crystal form B of the methanesulfonate of the compound of formula I, with a chemical purity of 99.68 area%. The XRPD results are shown in FIG. 5-1, and the characteristic peaks are shown in Table 9. The ¹H NMR (FIG. 5-3, DMSO-*d₆*) results indicate that the molar ratio of methanesulfonic acid to the compound of formula I in the sample was 1.0, the residual acetone solvent content was about 1.4 wt% (acetone:the compound of formula I = 0.2), and the residual EtOAc solvent content (contamination resulting from vacuum drying) was about 2.6 wt% (EtOAc:the compound of formula I = 0.2).

**Table 9. Analytical data of the XRPD pattern of the crystal form B of the methanesulfonate of the compound of formula I**

| No. | 2θ±0.2 (°) | Relative intensity (%) | No. | 2θ±0.2 (°) | Relative intensity (%) |
|---|---|---|---|---|---|
| 1 | 6.2955 | 75.43 | 11 | 18.3104 | 18.28 |
| 2 | 9.4469 | 84.75 | 12 | 18.7649 | 23.33 |
| 3 | 10.6946 | 58.42 | 13 | 19.4556 | 28.87 |
| 4 | 11.2857 | 100.00 | 14 | 19.9441 | 37.49 |
| 5 | 12.1034 | 22.70 | 15 | 20.9720 | 18.27 |
| 6 | 12.7182 | 14.07 | 16 | 21.6727 | 89.38 |
| 7 | 13.4232 | 12.51 | 17 | 24.6434 | 10.91 |
| 8 | 15.0752 | 44.64 | 18 | 27.7825 | 13.48 |
| 9 | 16.6835 | 65.23 | 19 | 28.6446 | 16.87 |
| 10 | 17.3063 | 15.47 | | | |

### Example 6. Benzenesulfonate of Compound of Formula I

The compound of formula I (25 mg) and benzenesulfonic acid (in an equimolar ratio) were weighed out, and 0.5-1.0 mL of ACN was added. After the mixture was magnetically stirred at room temperature for 2.5 days, cycles of heating and cooling (50 °C-20 °C-50 °C-20 °C, heating and cooling rate: 10 °C/h) were performed, and a solid was centrifugally separated and dried under vacuum at 40 °C for 3 h to give the crystal form A of the benzenesulfonate of the compound of formula I, with a chemical purity of 99.78 area%. The XRPD results (FIG. 6-1) indicate that the sample had relatively high crystallinity, and its characteristic peaks are shown in Table 10. The ¹H NMR (FIG. 6-2, DMSO-d6) results indicate that the molar ratio of benzenesulfonic acid to the compound of formula I in the sample was 0.8, and no additional solvent signals were observed. The TGA results (FIG. 6-3) show that the sample had a weight loss of 1.7% at 150 °C, which corresponds to about 0.8 H₂O molecules. The crystal form A of the benzenesulfonate was a hydrate of a monobenzenesulfonate.

**Table 10. Analytical data of the XRPD pattern of the crystal form A of the benzenesulfonate of the compound of formula I**

| No. | 2θ±0.2 (°) | Relative intensity (%) | No. | 2θ±0.2 (°) | Relative intensity (%) |
|---|---|---|---|---|---|
| 1 | 5.5180 | 2.32 | 15 | 19.2128 | 20.46 |
| 2 | 8.4849 | 23.22 | 16 | 19.9372 | 22.11 |
| 3 | 8.8939 | 33.91 | 17 | 20.8604 | 100.00 |
| 4 | 9.1410 | 91.35 | 18 | 21.5615 | 22.81 |
| 5 | 10.3692 | 8.19 | 19 | 22.6032 | 6.76 |
| 6 | 11.2190 | 33.32 | 20 | 23.7113 | 35.17 |
| 7 | 11.9410 | 8.95 | 21 | 24.0194 | 29.55 |
| 8 | 14.2050 | 28.57 | 22 | 24.4098 | 15.98 |
| 9 | 14.3394 | 12.87 | 23 | 24.7649 | 23.02 |
| 10 | 14.5299 | 6.27 | 24 | 26.3811 | 13.06 |
| 11 | 15.6291 | 46.37 | 25 | 26.9739 | 12.69 |
| 12 | 16.5247 | 15.34 | 26 | 30.0137 | 15.31 |
| 13 | 17.4368 | 10.92 | 27 | 32.4860 | 2.22 |
| 14 | 18.4305 | 15.10 | | | |

### Example 7. Gentisate of Compound of Formula I

The compound of formula I (25 mg) and gentisic acid (in an equimolar ratio) were weighed out, and 0.5-1.0 mL of MeOH:DCM (1:1, v/v) was added. After the mixture was magnetically stirred at room temperature for 2.5 days, the mixture was stirred at 5 °C for 1 day and left to stand at -20 °C for 1 day. Toluene was added as an anti-solvent and volatilized at room temperature, and a solid was centrifugally separated and dried under vacuum at 40 °C for 3 h to give the crystal form A of the gentisate of the compound of formula I, with a chemical purity of 98.64 area%. The XRPD results (FIG. 7-1) show that the sample had relatively high crystallinity, and its characteristic peaks are shown in Table 11. According to the ¹H NMR (FIG. 7-2, DMSO-d6) spectrum, the residual toluene solvent content was about 1.2 wt% (toluene:the compound of formula I = 0.1). The TGA results (FIG. 7-3) indicate that the sample had a weight loss of 6.1% before 200 °C, which corresponds to about 2.2 H₂O molecules and 0.1 toluene molecules. The crystal form A of the gentisate was a hydrate.

**Table 11. Analytical data of the XRPD pattern of the crystal form A of the gentisate of the compound of formula I**

| No. | 2θ±0.2 (°) | Relative intensity (%) | No. | 2θ±0.2 (°) | Relative intensity (%) |
|---|---|---|---|---|---|
| 1 | 4.3974 | 1.25 | 14 | 21.2453 | 23.05 |
| 2 | 6.5498 | 81.08 | 15 | 21.9965 | 13.99 |
| 3 | 7.8186 | 50.28 | 16 | 22.1812 | 10.55 |
| 4 | 10.1932 | 100.00 | 17 | 22.5808 | 21.58 |
| 5 | 12.0696 | 51.36 | 18 | 23.1164 | 12.00 |
| 6 | 13.1877 | 26.83 | 19 | 24.3675 | 29.24 |
| 7 | 13.5227 | 9.13 | 20 | 24.8161 | 19.09 |
| 8 | 15.1362 | 20.99 | 21 | 25.8173 | 69.26 |
| 9 | 15.9457 | 14.13 | 22 | 26.6389 | 40.40 |
| 10 | 16.8632 | 14.81 | 23 | 27.7044 | 34.86 |
| 11 | 17.3902 | 24.75 | 24 | 28.9926 | 8.89 |
| 12 | 18.6218 | 79.85 | 25 | 31.7437 | 14.41 |
| 13 | 20.9685 | 48.17 | | | |

### Test Example 1. In Vitro Antagonistic Activity Screening

Cell: HEK293 cells stably expressing human GnRHR (WuXi AppTec (Shanghai) Co., Ltd.) Equipment: 384-well plate, Greiner; Vi-cell XR Cell Viability Analyzer, Beckman Coulter; incubator, Thermo.

Method: HEK293 cells stably expressing human GnRHR were seeded into a 384-well plate at a density of 20,000 cells/well. Fluo-4 DirectTM No-wash Loading Buffer (Invitrogen) was added to each well the next day, and then the plate was put back into an incubator, incubated at 37 °C for 50 min, and then incubated at room temperature for 10 min. The test compound was dissolved in DMSO, and 10 µL of the solution was added to each well; the test concentrations were 1000 nM, 250 nM, 62.5 nM, 15.6 nM, 3.9 nM, 976.6 pM, 244.1 pM, 61.0 pM, 15.3 pM, and 3.8 pM. Fluorescence was measured using a FLIPR Tetra high-throughput real-time fluorescence assay and analysis system. Then 10 µL of Leuprolide acetate was added, and fluorescence was measured. The IC₅₀ and IC₉₀ of the compound were calculated using Prism. The results are shown in Table 12.

**Table 12. The in vitro antagonistic activity of the compound of formula I against human GnRHR**

| Compound | Functional IC₅₀ (nM) | Functional IC₉₀ (nM) |
|---|---|---|
| Formula I | 1.01 | 7.55 |

Conclusion: The compound of formula I had a significant inhibitory effect on human GnRHR.

### Test Example 2. In Vitro Binding Screening

Cell: Chem-1 cells stably expressing human GnRHR (Eurofins Panlabs Discovery Services) Reagent: [¹²⁵I] [D-Trp6]-LH-RH, PerkinElmer; [D-Trp6]-LH-RH, Abcam.

Equipment: MicroBeta2 LumiJET system, PerkinElmer.

Method: A chem-1 cell membrane stably expressing human GnRHR was resuspended in HEPES (pH 7.4), and 6 µg of the human GnRHR cell membrane was added to each well and incubated with 0.05 nM [¹²⁵I]-[D-Trp6]-LH-RH and the test compound at 25 °C for 60 min. The compound was dissolved in DMSO; the test concentrations were 500 nM, 62.5 nM, 7.81 nM, 0.97 nM, 0.12 nM, 15 pM, and 1.9 pM. 1 µM [D-Trp6]-LH-RH was used in the non-specific binding assay. The cell membrane was collected by filtration under vacuum and washed before liquid scintillation. The binding IC₅₀ and IC₉₀ of the compound were calculated using Prism. The results are shown in Table 13.

**Table 13. The in vitro binding activity of the compound of the present disclosure to human GnRHR**

| Compound | Binding IC₅₀ (nM) | Binding IC₉₀ (nM) |
|---|---|---|
| Formula I | 0.64 | 2.67 |

Conclusion: The compound of formula I had a significant binding ability to human GnRHR.

### Test Example 3. hERG Test

### Cell: CHO cells stably expressing a human hERG potassium channel (WuXi AppTec (Shanghai) Co., Ltd.)

Reagents: physiological solution (PH = 7.4): 140 mM NaCl, 4 mM KCl, 2 mM CaCl₂, 1 mM MgCl₂, 5 mM glucose, 10 mM HEPES, osmolarity: about 298 mOsm; external solution (PH = 7.4): 80 mM NaCl, 4 mM KCl, 2 mM CaCl₂, 1 mM MgCl₂, 5 mM glucose, 60 mM NMDG, 10 mM HEPES, osmolarity: about 289 mOsm; and internal solution (PH = 7.4): 10 mM NaCl, 10 mM KCl, 110 mM KF, 10 mM HEPES, EGTA: 10 nM, osmolarity: about 280 mOsm.

Equipment: QPatch high-throughput automated patch clamp system, Sophion.

Method: CHO cells stably expressing a human hERG potassium channel were digested with TrypLE and resuspended in the physiological solution for later use. The single-cell high-impedance sealing and whole-cell mode formation processes were automatically completed by Qpatch. The cells were clamped at a voltage of -80 mV, then a voltage of -50 mV was applied for 80 ms to subtract (current) leakage, and a voltage of +20 mV was applied for 4800 ms to open the hERG channel. The voltage was then reduced to -50 mV and maintained for 5000 ms. This process was used for data collection and analysis. Finally, the voltage was returned to the clamping voltage (-80 mV, 1000 ms). This process was repeated every 20,000 ms. 40 µL of a solvent control (external solution + DMSO) was added first, and testing was performed for 300 ms to collect baseline data. Then solutions of the test compound with various concentrations (40 µL) were added, and testing was performed for no less than 300 ms. The compound was dissolved in DMSO; the test concentrations were 0.30 µM, 1.00 µM, 3.00 µM, 10.00 µM, and 30.00 µM. IC₅₀ data of the hERG test were obtained by analysis using DataControl, Excel2013, and Prism. The results are shown in Table 14.

**Table 14. The inhibitory effects of the compound of formula I on the human hERG potassium channel**

| Compound | hERG IC₅₀ (µM) |
|---|---|
| Formula I | 25.79 |

Conclusion: The compound of formula I had relatively low inhibitory activity against the human hERG potassium channel, indicating that the compound has very low cardiotoxicity.

### Test Example 5. Evaluation of Permeability of Test Substance and Transporter Substrate

### Cell: Caco-2 cells

Instruments: water purification instrument, ELGA LabWate; biosafety cabinet, NUAIRE; thermostatic CO₂ incubator, Thermo; microplate reader, PerkinElmer; LC-MS/MS, AB SCIEX.

Method: A Caco-2 monolayer cell model was used to determine the two-way permeability of the test substance and evaluate whether the test substance was transported via efflux by a transporter. In the experiment, Caco-2 cells were seeded into a 96-well cell culture plate and cultured for 28 consecutive days before a transport assay. The test substance was administered on two sides. After 120 min of incubation, samples of apical end A, basal end B, and the cell lysate were collected, and the content of the test substance in the samples was quantitatively determined using a liquid chromatography-tandem mass spectrometry (LC/MS/MS) method. The apparent permeability coefficients (Papp) in the direction from the apical end to the basal end (A-B) and in the direction from the basal end to the apical end (B-A) and the efflux ratio were calculated based on the concentration of the test substance. The results are shown in Table 15.

**Table 15. The permeability of the compound of formula I in Caco-2 cells**

| Compound | Mean _{Papp} (10⁻⁶ cm/s) | | Efflux ratio |
|---|---|---|---|
| | A to B | B to A | |
| Formula I | 0.0142 | 4.19 | 294 |

Conclusion: The compound of formula I had a relatively low efflux ratio.

### Test Example 6. Study on In Vivo Pharmacokinetics and Tissue Distribution in SD Rats

### Rats: SD rats, male, 200-250 g

Instruments: water purification instrument, Millipore; electronic balance, METTLER TOLEDO; highspeed benchtop centrifuge, Thermo; water bath thermostatic oscillator, Shanghai Yiheng Technical Co., Ltd.; vortex oscillator, Thermo; LC-MS/MS, AB SCIEX.

Method: Solutions of all compounds were prepared using DMSO and 20% solutol. In the pharmacokinetic experiment, the administration dose for the intravenous group (i.v) was 1 mg/kg, and the administration dose for the intragastric group (i.g) was 12 mg/kg; each group contained 3 rats. Blood was collected from the orbit of the rats before administration (0 h) and at set time points after administration. In the tissue distribution experimental group, the rats were intragastrically dosed at 12 mg/kg. After the administration, the rats were anesthetized and dissected at set time points, heart blood was collected, and heart perfusion and pituitary collection were performed, with 3 rats per time point. The plasma was placed in a heparinized EP tube and centrifuged at 13,500 rpm for 10 min to isolate the plasma. The pituitary was homogenized with water in a certain ratio. The plasma and pituitary homogenates were pre-treated and then analyzed by LC-MS/MS to determine the concentrations of the test substances in the plasma and the pituitary. The plasma drug concentration data were processed in a non-compartmental model using WinNonlin^{™} Version 6.3 (Pharsight, Mountain View, CA) pharmacokinetic software. The following pharmacokinetic parameters were calculated using a log-linear trapezoidal method: elimination phase half-life (T_{1/2}), mean residence time of drug *in vivo* from time point 0 to the last time point (MRT₀₋ₗₐₛₜ), area under the time-plasma concentration curve from time point 0 to the last time point (AUC₀₋ₗₐₛₜ), initial concentration (C₀) or maximum concentration Cₘₐₓ, and absolute bioavailability F. The results are shown in Tables 16-1, 16-2, and 16-3.

**Table 16-1. Pharmacokinetic parameters in SD rats after intravenous administration**

| Dose | Compound | T_{1/2} (h) | C₀ (ng/mL) | AUCₗₐₛₜ (h*ng/mL) | MRTₗₐₛₜ (h) |
|---|---|---|---|---|---|
| i.v 1 mg/kg | Relugolix | 3.64±1.25 | 339±36 | 212±61 | 2.01±0.92 |
| | Formula I | 6.39±1.08 | 68.8±21.7 | 150±12 | 5.41±0.47 |

**Table 16-2. Pharmacokinetic parameters in SD rats after intragastric administration**

| Dose | Compound | T_{1/2} (h) | Tₘₐₓ (h) | Cₘₐₓ (ng/mL) | AUCₗₐₛₜ (h*ng/mL) | MRTₗₐₛₜ (h) | F (%) |
|---|---|---|---|---|---|---|---|
| i.g 12 mg/kg | Relugolix | 3.88±0.57 | 0.330±0.140 | 21.6±8.3 | 146±37 | 5.68±0.17 | 5.74 |
| | Formula I | 4.31±1.81 | 0.38±0.14 | 78.5±36.4 | 357±85 | 6.13±1.10 | 19.9 |

**Table 16-3. Pituitary tissue distribution in SD rats after intragastric administration (12 mg/kg)**

| Compound | Time | Plasma | Pituitary | Pituitary/plasma |
|---|---|---|---|---|
| Relugolix | 1 h | 22.2±10.2 | 944±591 | 42.4 |
| | 4h | 16.3±5.52 | 1246±777 | 76.3 |
| | 12 h | 3.17±2.53 | 510±213 | 161 |
| Formula I | 1 h | 39.5±14.0 | 2250±474 | 56.9 |
| | 4 h | 22.8±2.5 | 1841±636 | 80.8 |
| | 12 h | 9.70±3.10 | 1254±494 | 129 |

Conclusion: In the intragastric administration group, the Cₘₐₓ, AUCₗₐₛₜ, and absolute bioavailability of the compound of formula I were 3.63 times, 2.45 times, and 3.47 times higher than those of Relugolix, respectively. The concentrations of the compound of formula I in the pituitary at 1 h, 4 h, and 12 h were 2.38 times, 1.48 times, and 2.46 times higher than those of Relugolix, respectively.

### Test Example 7. Suspension Competition Test of Crystal Forms A and B of Fumarate of Compound of Formula I

An excessive amount of the crystal form A of the fumarate of the compound of formula I was weighed into an HPLC vial, and 0.4 mL of MeOH was added. The mixture was magnetically stirred at room temperature for about 1.5 h and filtered through a nylon membrane (pore size: 0.22 µm) to give a saturated solution. About 1 mg of each of the crystal forms A and B of the fumarate of the compound of formula I was added to the saturated filtrate. The resulting mixture was magnetically stirred at room temperature for about 2.5 days, and a solid was centrifugally separated and subjected to XRPD testing. The results are shown in FIG. 8.

The results indicate that the obtained solid was the crystal form A of the fumarate of the compound of formula I, suggesting that the crystal form A of the fumarate of the compound of formula I is more stable at room temperature.

## Claims

1. A crystal form A of the compound of formula I, having, in an X-ray powder diffraction pattern using Cu-Kα radiation, characteristic diffraction peaks at the following 2θ angles: 7.2390 ± 0.2°, 8.7232 ± 0.2°, 10.5072 ± 0.2°, 12.4670 ± 0.2°, 18.6162 ± 0.2°, 19.6161 ± 0.2°, and 22.1387 ± 0.2°; preferably, the crystal form A has characteristic diffraction peaks at the following 2θ angles: 7.2390 ± 0.2°, 8.7232 ± 0.2°, 10.5072 ± 0.2°, 10.9392 ± 0.2°, 12.4670 ± 0.2°, 17.6527 ± 0.2°, 18.6162 ± 0.2°, 19.6161 ± 0.2°, 22.1387 ± 0.2°, 23.4047 ± 0.2°, and 27.3319 ± 0.2°; more preferably, the X-ray powder diffraction pattern using Cu-Kα radiation is shown in FIG. 1-1,

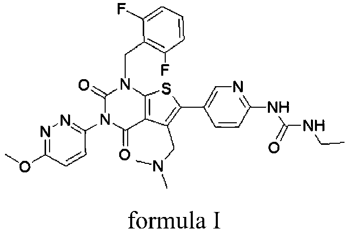

2. A pharmaceutically acceptable salt of the compound of formula I, wherein the pharmaceutically acceptable salt is selected from the group consisting of a hydrochloride, sulfate, phosphate, maleate, tartrate, fumarate, citrate, malate, hippurate, lactate, succinate, acetate, p-toluenesulfonate, methanesulfonate, benzenesulfonate, oxalate, malonate, gentisate, or benzoate; preferably a hydrochloride, fumarate, L-malate, methanesulfonate, benzenesulfonate, or gentisate.

3. A crystal form A of a fumarate of the compound of formula I, having, in an X-ray powder diffraction pattern using Cu-Kα radiation, characteristic diffraction peaks at the following 2θ angles: 6.9355 ± 0.2°, 9.6549 ± 0.2°, 14.4518 ± 0.2°, 18.8612 ± 0.2°, 20.2529 ± 0.2°, and 22.9352 ± 0.2°; preferably, the crystal form A has characteristic diffraction peaks at the following 2θ angles: 6.9355 ± 0.2°, 9.3706 ± 0.2°, 9.6549 ± 0.2°, 9.8505 ± 0.2°, 12.4723 ± 0.2°, 14.4518 ± 0.2°, 17.2206 ± 0.2°, 18.8612 ± 0.2°, 20.2529 ± 0.2°, 20.5609 ± 0.2°, 21.1589 ± 0.2°, 22.2396 ± 0.2°, 22.5989 ± 0.2°, 22.9352 ± 0.2°, and 26.2998 ± 0.2°; more preferably, the X-ray powder diffraction pattern using Cu-Kα radiation is shown in FIG. 2-1.

4. A crystal form B of a fumarate of the compound of formula I, having, in an X-ray powder diffraction pattern using Cu-Kα radiation, characteristic diffraction peaks at the following 2θ angles: 7.4623 ± 0.2°, 8.4044 ± 0.2°, 11.0463 ± 0.2°, 13.7771 ± 0.2°, 14.5639 ± 0.2°, and 23.0754 ± 0.2°; preferably, the crystal form B has characteristic diffraction peaks at the following 2θ angles: 7.4623 ± 0.2°, 8.4044 ± 0.2°, 11.0463 ± 0.2°, 13.7771 ± 0.2°, 14.5639 ± 0.2°, 17.6799 ± 0.2°, 19.5247 ± 0.2°, 20.9439 ± 0.2°, 21.1012 ± 0.2°, 23.0754 ± 0.2°, 24.1136 ± 0.2°, and 25.8680 ± 0.2°; more preferably, the X-ray powder diffraction pattern using Cu-Kα radiation is shown in FIG. 2-4.

5. A crystal form A of an L-malate of the compound of formula I, having, in an X-ray powder diffraction pattern using Cu-Kα radiation, characteristic diffraction peaks at the following 2θ angles: 9.2046 ± 0.2°, 9.6751 ± 0.2°, 12.4714 ± 0.2°, 13.5073 ± 0.2°, 17.1769 ± 0.2°, 20.1444 ± 0.2°, and 22.8785 ± 0.2°; preferably, the crystal form A has characteristic diffraction peaks at the following 2θ angles: 9.2046 ± 0.2°, 9.6751 ± 0.2°, 12.4714 ± 0.2°, 13.5073 ± 0.2°, 14.4312 ± 0.2°, 17.1769 ± 0.2°, 18.0032 ± 0.2°, 18.6031 ± 0.2°, 18.8495 ± 0.2°, 20.1444 ± 0.2°, 20.6407 ± 0.2°, 22.2793 ± 0.2°, 22.8785 ± 0.2°, 23.1632 ± 0.2°, 26.2217 ± 0.2°, and 27.0313 ± 0.2°; more preferably, the X-ray powder diffraction pattern using Cu-Kα radiation is shown in FIG. 3-1.

6. A crystal form A of a hydrochloride of the compound of formula I, having, in an X-ray powder diffraction pattern using Cu-Kα radiation, characteristic diffraction peaks at the following 2θ angles: 9.1252 ± 0.2°, 10.4351 ± 0.2°, 11.2797 ± 0.2°, and 12.9108 ± 0.2°; preferably, the crystal form A has characteristic diffraction peaks at the following 2θ angles: 9.1252 ± 0.2°, 9.6045 ± 0.2°, 10.4351 ± 0.2°, 11.2797 ± 0.2°, 12.9108 ± 0.2°, 17.0513 ± 0.2°, 17.5138 ± 0.2°, 20.9172 ± 0.2°, 21.6060 ± 0.2°, and 26.0138 ± 0.2°; more preferably, the X-ray powder diffraction pattern using Cu-Kα radiation is shown in FIG. 4-1.

7. A crystal form B of a hydrochloride of the compound of formula I, having, in an X-ray powder diffraction pattern using Cu-Kα radiation, characteristic diffraction peaks at the following 2θ angles: 9.4060 ± 0.2°, 10.0584 ± 0.2°, 12.0366 ± 0.2°, 14.9457 ± 0.2°, 17.3666 ± 0.2°, and 24.9835 ± 0.2°; preferably, the crystal form B has characteristic diffraction peaks at the following 2θ angles: 9.4060 ± 0.2°, 10.0584 ± 0.2°, 12.0366 ± 0.2°, 13.2712 ± 0.2°, 14.9457 ± 0.2°, 17.3666 ± 0.2°, 17.5949 ± 0.2°, 23.6400 ± 0.2°, 23.8466 ± 0.2°, 24.9835 ± 0.2°, and 27.2387 ± 0.2°; more preferably, the X-ray powder diffraction pattern using Cu-Kα radiation is shown in FIG. 4-1.

8. A crystal form C of a hydrochloride of the compound of formula I, having, in an X-ray powder diffraction pattern using Cu-Kα radiation, characteristic diffraction peaks at the following 2θ angles: 8.3817 ± 0.2°, 10.6700 ± 0.2°, 11.3268 ± 0.2°, and 16.1577 ± 0.2°; preferably, the crystal form C has characteristic diffraction peaks at the following 2θ angles: 8.3817 ± 0.2°, 10.6700 ± 0.2°, 11.3268 ± 0.2°, 16.1577 ± 0.2°, 16.9678 ± 0.2°, 18.5478 ± 0.2°, and 21.5630 ± 0.2°; more preferably, the X-ray powder diffraction pattern using Cu-Kα radiation is shown in FIG. 4-1.

9. A crystal form A of a methanesulfonate of the compound of formula I, having, in an X-ray powder diffraction pattern using Cu-Kα radiation, characteristic diffraction peaks at the following 2θ angles: 6.6779 ± 0.2°, 7.2085 ± 0.2°, 9.9783 ± 0.2°, and 10.5584 ± 0.2°; preferably, the crystal form A has characteristic diffraction peaks at the following 20 angles: 6.6779 ± 0.2°, 7.2085 ± 0.2°, 9.9783 ± 0.2°, 10.5584 ± 0.2°, 15.3689 ± 0.2°, 17.0799 ± 0.2°, 19.7556 ± 0.2°, and 21.0763 ± 0.2°; more preferably, the X-ray powder diffraction pattern using Cu-Kα radiation is shown in FIG. 5-1.

10. A crystal form B of a methanesulfonate of the compound of formula I, having, in an X-ray powder diffraction pattern using Cu-Kα radiation, characteristic diffraction peaks at the following 2θ angles: 6.2955 ± 0.2°, 9.4469 ± 0.2°, 10.6946 ± 0.2°, 11.2857 ± 0.2°, 16.6835 ± 0.2°, and 21.6727 ± 0.2°; preferably, the crystal form B has characteristic diffraction peaks at the following 2θ angles: 6.2955 ± 0.2°, 9.4469 ± 0.2°, 10.6946 ± 0.2°, 11.2857 ± 0.2°, 12.1034 ± 0.2°, 15.0752 ± 0.2°, 16.6835 ± 0.2°, 18.7649 ± 0.2°, 19.4556 ± 0.2°, 19.9441 ± 0.2°, and 21.6727 ± 0.2°; more preferably, the X-ray powder diffraction pattern using Cu-Kα radiation is shown in FIG. 5-1.

11. A crystal form A of a benzenesulfonate of the compound of formula I, having, in an X-ray powder diffraction pattern using Cu-Kα radiation, characteristic diffraction peaks at the following 2θ angles: 8.4849 ± 0.2°, 8.8939 ± 0.2°, 9.1410 ± 0.2°, 11.2190 ± 0.2°, 14.2050 ± 0.2°, 15.6291 ± 0.2°, 20.8604 ± 0.2°, and 23.7113 ± 0.2°; preferably, the crystal form A has characteristic diffraction peaks at the following 2θ angles: 8.4849 ± 0.2°, 8.8939 ± 0.2°, 9.1410 ± 0.2°, 11.2190 ± 0.2°, 14.2050 ± 0.2°, 15.6291 ± 0.2°, 19.2128 ± 0.2°, 19.9372 ± 0.2°, 20.8604 ± 0.2°, 21.5615 ± 0.2°, 23.7113 ± 0.2°, 24.0194 ± 0.2°, and 24.7649 ± 0.2°; more preferably, the X-ray powder diffraction pattern using Cu-Kα radiation is shown in FIG. 6-1.

12. A crystal form A of a gentisate of the compound of formula I, having, in an X-ray powder diffraction pattern using Cu-Kα radiation, characteristic diffraction peaks at the following 2θ angles: 6.5498 ± 0.2°, 7.8186 ± 0.2°, 10.1923 ± 0.2°, 12.0696 ± 0.2°, 18.6218 ± 0.2°, and 25.8173 ± 0.2°; preferably, the crystal form A has characteristic diffraction peaks at the following 2θ angles: 6.5498 ± 0.2°, 7.8186 ± 0.2°, 10.1923 ± 0.2°, 12.0696 ± 0.2°, 13.1877 ± 0.2°, 15.1362 ± 0.2°, 17.3902 ± 0.2°, 18.6218 ± 0.2°, 20.9685 ± 0.2°, 21.2453 ± 0.2°, 24.3675 ± 0.2°, 25.8173 ± 0.2°, 26.6389 ± 0.2°, and 27.7044 ± 0.2°; more preferably, the X-ray powder diffraction pattern using Cu-Kα radiation is shown in FIG. 7-1.

13. A pharmaceutical composition, comprising the crystal form according to any one of claims 1 and 3-12 or the pharmaceutically acceptable salt according to claim 2, and a pharmaceutically acceptable carrier.

14. Use of the crystal form according to any one of claims 1 and 3-12, the pharmaceutically acceptable salt according to claim 2, or the pharmaceutical composition according to claim 13 in the preparation of a medicament for preventing and/or treating a sex hormone-dependent disease.

15. Use of the crystal form according to any one of claims 1 and 3-12, the pharmaceutically acceptable salt according to claim 2, or the pharmaceutical composition according to claim 13 in the preparation of a medicament for preventing and/or treating a disease of sex hormone-dependent cancer, bone metastasis of sex hormone-dependent cancer, prostatic hypertrophy, hysteromyoma, endometriosis, metrofibroma, precocious puberty, amenorrhea, premenstrual syndrome, dysmenorrhoea, multilocular ovarian syndrome, polycystic ovary syndrome, acne, alopecia, Alzheimer's disease, infertility, irritable bowel syndrome, benign or malignant tumors that are hormone-independent and sensitive to luteinizing hormone-releasing hormone, or flushing, or as a reproduction modulator, a contraceptive drug, an ovulation inducer, or a medicament for preventing early ovulation, or as a medicament for preventing a postoperative recurrence of sex hormone-dependent cancer.
